# EUROPEAN PATENT APPLICATION

(11) **EP 4 331 476 A1**
(43) Date of publication of application: **06.03.2024**
(21) Application number: 23194283.0
(22) Date of filing: 30.08.2023
(51) Int. Cl.: A61B 5/0205, A61H 31/00, A61N 1/39, A61B 5/318, A61B 5/361

(54) **ESTABLISHING AND CEASING COMMUNICATION BETWEEN MEDICAL DEVICES**

(30) Priority: 31.08.2022 US 202263402805 P
(71) Applicant: Stryker Corporation, Kalamazoo, MI 49002 (US)
(72) Inventor: GREENEWALD, Robert, Kalamazoo, 49002 (US); APPERSON, Ryan W., Kalamazoo, 49002 (US); CHAPMAN, Fred W., Kalamazoo, 49002 (US); LAGERSTROM, Jonas, Kalamazoo, 49002 (US); LINDROTH, Sara, Kalamazoo, 49002 (US); RUTZER, Mark, Kalamazoo, 49002 (US); SANDRUP, Eric, Kalamazoo, 49002 (US); SVAHN, Tobias, Kalamazoo, 49002 (US)
(74) Representative: V.O.

(57) **Abstract**

Techniques for pairing medical devices are described. An example method includes detecting a signal indicating a physiological parameter of a subject and determining, by analyzing the signal, that a second medical device is administering a treatment to the subject. In response to determining that the second medical device is administering the treatment to the subject, the method further includes pairing a first medical device with the second medical device. In response to pairing the first medical device with the second medical device, the method further includes transmitting data to the second medical device over a wireless channel.

## Description

### BACKGROUND

Medical devices can be used to facilitate patient monitoring, facilitate patient treatments, or a combination of both. In some environments, multiple medical devices are located in close proximity to one another. Some may be monitoring and/or treating the same patient, and some may be monitoring and/or treating different patients.

### SUMMARY

According to an aspect is provided a first medical device, comprising a sensor configured to detect a parameter signal indicating a physiological parameter of a subject; a transceiver; and a processor. The processor is configured to determine, by analyzing the parameter signal, that a second medical device is administering a treatment to the subject. The processor is configured to, in response to determining that the second medical device is administering the treatment to the subject, pair the first medical device with the second medical device. The processor is configured to, in response to pairing the first medical device with the second medical device, cause the transceiver to transmit, over a wireless channel, data to the second medical device or to receive, over the wireless channel, data from the second medical device.

Optionally, the sensor comprises an electrocardiogram (ECG) sensor or an impedance sensor, and the treatment comprises chest compressions.

Optionally, the processor is configured to determine, by analyzing the parameter signal, that the second medical device is administering the treatment to the subject by detecting, in the parameter signal, an artifact indicative of the chest compressions being administered to the subject by the second medical device.

Optionally, the first medical device further comprises an input device configured to receive an input signal indicating the second medical device, wherein the processor is configured to pair the first device with the second medical device in response to the input device receiving the input signal.

Optionally, the transceiver is configured to receive a signal that comprises pairing information, and the processor is configured to pair the first device with the second device by utilizing the pairing information. Optionally, the transceiver receives the signal from the second medical device, a passkey device, a cot, or an accessory device.

Optionally, the transceiver is configured to receive, from the second medical device over the wireless channel, a signal that indicates a treatment parameter, and the processor is configured to determine, by analyzing the parameter signal, that the second medical device is administering the treatment to the subject by determining, by analyzing the parameter signal, that the treatment is consistent with the treatment parameter.

Optionally, the treatment is a first treatment, the first medical device further comprising memory storing clock data. The processor can be configured to cause the transceiver to transmit, to the second medical device over the wireless channel, a synchronization message indicative of the clock data; and cause the transceiver to transmit, to the second medical device over the wireless channel, an instruction to perform a second treatment at a time that is in reference to the clock data.

Optionally, the treatment is a first treatment, and the processor is configured to cause the transceiver to periodically transmit, to the second medical device over the wireless channel, first data packets indicating the physiological parameter of the subject; and cause the transceiver to transmit, to the second medical device over the wireless channel, a second data packet comprising an instruction to perform a second treatment.

Optionally, the transceiver configured to receive a wireless signal from the second medical device over a communication channel; and the processor is configured to: determine that the subject is not receiving the treatment during a second time period by determining that an artifact associated with the treatment is omitted from the parameter signal during the second time period; and in response to determining that the subject is not receiving the treatment during the second time period, causing the transceiver to end the communication channel.

According to an aspect is provided a method, comprising detecting a signal indicating a physiological parameter of a subject; determining, by analyzing the signal, that a second medical device is administering a treatment to the subject; in response to determining that the second medical device is administering the treatment to the subject, pairing a first medical device with the second medical device; and in response to pairing the first medical device with the second medical device, transmitting data to the second medical device over a wireless channel. The first medical device can e.g. be the first medical device described hereinabove. The first medical device can e.g. be a monitor-defibrillator. The second medical device can e.g. be a mechanical chest compression device.

Optionally, the physiological parameter comprises an electrocardiogram (ECG), an impedance, a force, a blood pressure, a partial pressure of carbon dioxide, a partial pressure of oxygen, a capnograph, an end tidal gas parameter, a flow rate, a blood oxygenation, an EEG, a temperature, a heart sound, a blood flow rate, a physiological geometry, a heart rate, or a pulse rate of the subject.

Optionally, transmitting the data to the second medical device over the wireless channel comprises transmitting an electromagnetic signal encoded with the data.

Optionally, determining, by analyzing the signal, that the second medical device is administering the treatment to the subject comprises detecting, in the signal, an artifact indicative of the treatment being administered to the subject by the second medical device.

Optionally, the signal is a first signal, and the method further comprises receiving a second signal comprising pairing information, wherein pairing the first medical device with the second device comprises identifying a link key based on the pairing information, and wherein transmitting data to the second medical device over the wireless channel comprises encrypting the data using the link key. Optionally, the second signal is received from the second medical device, a passkey device, a cot, or an accessory device.

Optionally, the signal is a first signal, and the method further comprises receiving a second signal comprising a treatment parameter, wherein determining, by analyzing the first signal, that the second medical device is administering the treatment to the subject comprises determining, by analyzing the signal, that the treatment is consistent with the treatment parameter.

Optionally, the treatment is a first treatment, and transmitting data over the wireless channel comprises periodically transmitting, to the second medical device over the wireless channel, first data packets indicating the physiological parameter of the subject; and transmitting, to the second medical device over the wireless channel, a second data packet comprising an instruction to perform a second treatment.

According to an aspect is provided a system, comprising: a mechanical chest compression device configured to administer chest compressions to a subject and to broadcast a first wireless signal indicating a frequency or a timing of the chest compressions and a first medical device as described hereinabove. The first medical device can comprises a monitor-defibrillator. The sensor can comprise a detection circuit configured to detect a parameter signal comprising an electrocardiogram (ECG) of the subject. The transceiver can be configured to receive the first wireless signal from the mechanical chest compression device and to transmit a second wireless signal to the mechanical chest compression device. The processor can be configured to determine that the mechanical chest compression device is administering the chest compressions to the subject by analyzing the parameter signal in view of the frequency or the timing of the chest compressions; in response to determining that the mechanical chest compression device is administering the chest compressions to the subject, cause the transceiver to initiate a wireless communication channel with the mechanical chest compression device; determine that the subject is experiencing ventricular fibrillation (VF) by analyzing the parameter signal; and in response to determining that the subject is experiencing VF cause the transceiver to transmit the second wireless signal to the mechanical chest compression device over the wireless communication channel, the second wireless signal comprising an instruction to cease administering the chest compressions at a predetermined time. The monitor-defibrillator comprises a discharge circuit configured to output an electrical shock to the subject. The processor can be configured to cause the discharge circuit to output the electrical shock to the subject at the predetermined time.

According to an aspect is provided a system, comprising a mechanical chest compression device configured to administer chest compressions to a subject and to broadcast a first wireless signal indicating a frequency or a timing of the chest compressions; and a monitor-defibrillator. The monitor defibrillator comprises a detection circuit configured to detect a parameter signal comprising an electrocardiogram (ECG) of the subject; a transceiver configured to receive the first wireless signal from the mechanical chest compression device and to transmit a second wireless signal to the mechanical chest compression device; a discharge circuit configured to output an electrical shock to the subject; and a processor configured to: determine that the mechanical chest compression device is administering the chest compressions to the subject by analyzing the parameter signal in view of the frequency or the timing of the chest compressions; in response to determining that the mechanical chest compression device is administering the chest compressions to the subject, cause the transceiver to initiate a wireless communication channel with the mechanical chest compression device; determine that the subject is experiencing ventricular fibrillation (VF) by analyzing the parameter signal; and in response to determining that the subject is experiencing VF: cause the transceiver to transmit the second wireless signal to the mechanical chest compression device over the wireless communication channel, the second wireless signal comprising an instruction to cease administering the chest compressions at a predetermined time; and cause the discharge circuit to output the electrical shock to the subject at the predetermined time.

Optionally, the processor is further configured to identify a comb filter characterized by the frequency or the timing of the chest compressions; remove a chest compression artifact from data representing the parameter signal by applying the comb filter to the data, and determining that the subject is experiencing VF by analyzing the parameter signal comprises analyzing the data representing the parameter signal in response to applying the comb filter to the data.

Optionally, the first wireless signal further comprises an identifier of the mechanical chest compression device and a communication capability of the mechanical chest compression device, and the processor is configured to cause the transceiver to initiate the wireless communication channel with the mechanical chest compression device by: causing the transceiver to transmit, to the mechanical chest compression device, a pairing request comprising the identifier, the pairing request being consistent with the communication capability of the mechanical chest compression device; and causing the transceiver to receive, from the mechanical chest compression device, a pairing response indicating that the wireless communication channel has been established.

According to an aspect is provided a first medical device, e.g. the first medical device described hereinabove, including: a sensor configured to detect a parameter signal indicating a physiological parameter of a subject, the subject receiving a treatment from a second medical device during a first time period; a transceiver configured to receive a wireless signal from the second medical device over a communication channel; and a processor configured to: determine that the subject is not receiving the treatment during a second time period by determining that an artifact associated with the treatment is omitted from the parameter signal during the second time period; and in response to determining that the subject is not receiving the treatment during the second time period, causing the transceiver to end the communication channel.

Optionally, the sensor includes an electrocardiogram (ECG) sensor or an impedance sensor.

Optionally, the treatment includes chest compressions, pacing, or an electrical shock.

Optionally, the treatment is a first treatment, and the wireless signal indicates a treatment parameter characterizing the first treatment administered by the second medical device, and wherein the processor is further configured to: generate a filter characterized by the treatment parameter; remove an artifact from data representing the parameter signal during the first time period by applying the filter to the data; in response to removing the artifact from the data representing the parameter signal during the first time period, generate a recommendation for a second treatment by analyzing the data representing the parameter signal during the first time period; and output the recommendation. Optionally, the recommendation is to administer an electrical shock, administer chest compressions, or pause the chest compressions. Optionally, the recommendation is a first recommendation, the data is first data, and the processor is further configured to: generate a second recommendation for a third treatment by analyzing second data representing the parameter signal during the second time period without applying the filter to the second data; and output the second recommendation.

Optionally, the wireless signal indicates that the second medical device is administering the treatment during the first time period and the second time period.

Optionally, the first medical device includes a mechanical chest compression device or an external defibrillator.

Optionally, the wireless signal is a first wireless signal, and the processor is configured to cause the transceiver to end the communication channel by: causing the transceiver to transmit, to the second medical device, a second wireless signal indicating an unpairing instruction.

According to an aspect is provided a method, e.g. the method described hereinabove, performed by a first medical device, e.g. the first medical device described hereinabove, the method including: detecting a parameter signal indicating a physiological parameter of a subject, the subject receiving a treatment from a second medical device during a first time period; receiving a wireless signal from the second medical device over a communication channel; determining that the subject is not receiving the treatment during a second time period by determining that an artifact associated with the treatment is omitted from the parameter signal during the second time period; and in response to determining that the subject is not receiving the treatment during the second time period, ending the communication channel.

Optionally, the physiological parameter includes an electrocardiogram (ECG) or an impedance.

Optionally, the treatment includes chest compressions, an electrical shock, or pacing.

Optionally, the treatment is a first treatment, and the wireless signal indicates a treatment parameter characterizing the first treatment administered by the second medical device, and the method further includes: generating a filter characterized by the treatment parameter; removing an artifact from data representing the parameter signal during the first time period by applying the filter to the data; in response to removing the artifact from the data representing the parameter signal during the first time period, generating a recommendation for a second treatment by analyzing the data representing the parameter signal during the first time period; and outputting the recommendation. Optionally, the recommendation is to administer an electrical shock, administer chest compressions, or pause the chest compressions. Optionally, the recommendation is a first recommendation, the data is first data, and the method further includes: generating a second recommendation for a third treatment by analyzing second data representing the parameter signal during the second time period without applying the filter to the second data; and outputting the second recommendation.

Optionally, the wireless signal indicates that the second medical device is administering the treatment during the first time period and the second time period.

Optionally, the wireless signal is a first wireless signal, and ending the communication channel includes: transmitting, to the second medical device, a second wireless signal indicating an unpairing instruction.

According to an aspect is provided a system including: a mechanical chest compression device configured to administer compressions, to broadcast a first wireless signal including a treatment parameter characterizing the compressions, and to receive a second wireless signal over a communication channel; and a first medical device as described hereinabove. The first medical device can comprise a monitor-defibrillator. The monitor-defibrillator can include a detection circuit configured to detect a parameter signal including an electrocardiogram (ECG) of a subject. The transceiver can be configured to receive the first wireless signal from the mechanical chest compression device and to transmit the second wireless signal to the mechanical chest compression device over the communication channel. The monitor defibrillator can comprise a discharge circuit configured to output an electrical shock to the subject. The processor can be configured to: identify a filter characterized by the treatment parameter; remove a compression artifact from first data representing the parameter signal detected during a first time period by applying the filter to the first data; determine that the compression artifact is omitted from the parameter signal during a second time period; determine that the treatment parameter indicates that the mechanical chest compression device is administering the compressions during the second time period; and in response to determining that the compression artifact is omitted from the parameter signal during the second time period and to determining that the treatment parameter indicates that the mechanical chest compression device is administering the compressions during the second time period: cause the transceiver to end the communication channel with the mechanical chest compression device; refrain from applying the filter to second data representing the parameter signal detected during the second time period; determine that the subject is experiencing ventricular fibrillation (VF) by analyzing second data; and in response to determining that the subject is experiencing VF, cause the discharge circuit to output the electrical shock to the subject.

According to an aspect is provided a system, e.g. a system as described hereinabove, including: a mechanical chest compression device configured to administer compressions, to broadcast a first wireless signal including a treatment parameter characterizing the compressions, and to receive a second wireless signal over a communication channel; and a monitor-defibrillator. The monitor-defibrillator includes: a detection circuit configured to detect a parameter signal including an electrocardiogram (ECG) of a subject; a transceiver configured to receive the first wireless signal from the mechanical chest compression device and to transmit the second wireless signal to the mechanical chest compression device over the communication channel; a discharge circuit configured to output an electrical shock to the subject; and a processor configured to: identify a filter characterized by the treatment parameter; remove a compression artifact from first data representing the parameter signal detected during a first time period by applying the filter to the first data; determine that the compression artifact is omitted from the parameter signal during a second time period; determine that the treatment parameter indicates that the mechanical chest compression device is administering the compressions during the second time period; and in response to determining that the compression artifact is omitted from the parameter signal during the second time period and to determining that the treatment parameter indicates that the mechanical chest compression device is administering the compressions during the second time period: cause the transceiver to end the communication channel with the mechanical chest compression device; refrain from applying the filter to second data representing the parameter signal detected during the second time period; determine that the subject is experiencing ventricular fibrillation (VF) by analyzing second data; and in response to determining that the subject is experiencing VF, cause the discharge circuit to output the electrical shock to the subject.

Optionally, the treatment parameter indicates a frequency of the compressions, and the filter includes a comb filter including a first notch at the frequency of the compressions and a second notch at a harmonic of the frequency of the compressions.

Optionally, the processor is configured to cause the transceiver to end the communication channel with the mechanical chest compression device by: causing the transceiver to transmit a third wireless signal to the mechanical chest compression device, the third wireless signal indicating an unpairing instruction.

It will be appreciated that any of the aspects, features and options described herein can be combined. It will particularly be appreciated that any of the aspects, features and options described in view of the devices apply equally to the methods and systems, and vice versa.

### BRIEF DESCRIPTION OF THE DRAWINGS

FIGS. 1A and 1B illustrate example environments for establishing communications between medical devices at a rescue scene.
FIG. 2 illustrates an example environment in which a monitoring device and a treating device are configured to communicate via one or more connections.
FIG. 3 illustrates example signaling for facilitating communication between medical devices.
FIG. 4 illustrates example signaling for ceasing communication between medical devices.
FIG. 5 illustrates an example environment of interconnected devices that facilitate medical device pairing.
FIG. 6 illustrates an example process for pairing medical devices.
FIG. 7 illustrates an example process for unpairing medical devices.
FIG. 8 illustrates an example of an external defibrillator configured to perform various functions described herein.
FIG. 9 illustrates a chest compression device configured to perform various functions described herein.

### DETAILED DESCRIPTION

Various implementations described herein relate to techniques for establishing and ceasing communication between medical devices. In some examples, multiple medical devices are managing care of the same patient. For instance, a monitoring device detects a condition of the patient and a treating device administers a treatment to the patient. The treatment, for example, is enhanced when the devices coordinate with one another. In particular examples, the devices coordinate with each other by exchanging messages over a wireless interface. The messages, for instance, include reports about the condition of the patient, reports about ongoing treatments to the patient, as well as instructions to perform actions (e.g., additional treatments).

However, particular environments include numerous medical devices capable of wireless communication. It is therefore important that a given medical device communicate with another medical device monitoring and/or treating the same patient, rather than other medical devices, which may be treating other patients. In addition, in high-stress clinical environments, users may be busy with patient care and unable to perform complex tasks for establishing pairing between devices. It is therefore preferred, in some environments, that medical devices are able to pair with each other with minimal user intervention.

According to various implementations of the present disclosure, the monitoring device identifies the treating device by analyzing a parameter signal detected from the patient. The parameter signal, for instance, indicates a physiological parameter (e.g., an electrocardiogram (ECG)) of the patient, and includes an artifact that is indicative of the treatment being administered to the patient (e.g., chest compressions) by the treating device. The monitoring device determines a characteristic of the treatment (e.g., a frequency of the chest compressions) by analyzing the parameter signal. When the monitoring device receives a pairing request from the treating device that further indicates the characteristic of the treatment, the monitoring device can confirm that the pairing request is from the device treating the patient. Accordingly, the monitoring device can automatically pair with the treating device based on the pairing request without a risk of pairing to an incorrect device (e.g., a device treating a different patient). However, if the monitoring device receives a pairing request from another treating device that does not indicate the characteristic of the treating, the monitoring device refrains from pairing to that device. Thus, implementations of the present disclosure enable the monitoring device to accurately pair to one or more devices associated with the same patient.

Similarly, in some cases, the monitoring device determines to unpair from the treating device by analyzing the parameter signal. If, for instance, the artifact indicative of the treatment is missing from the parameter signal, the monitoring device determines that the treating device is no longer treating the patient. In some cases, the monitoring device automatically unpairs from the treating device upon determining that the treating device is no longer treating the patient. Other techniques for accurately and/or efficiently pairing or unpairing medical devices are also within the scope of the present disclosure.

Implementations of the present disclosure are directed to improvements in the technical field of medical devices. Rather than requiring complex user interfaces and user interventions, implementations described herein enable automated and accurate pairing between medical devices with minimal to no user intervention. In some cases, numerous medical devices associated with the same patient can be paired together into a cohesive mesh, particularly in emergency settings where users are unable to manually pair individual devices together.

Various examples will now be described with reference to the accompanying drawings.

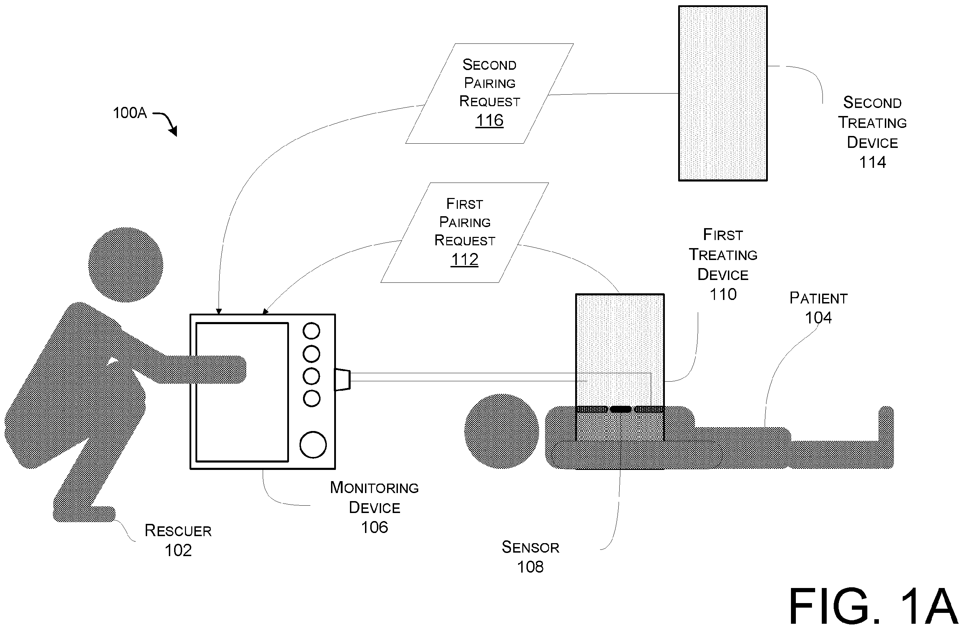

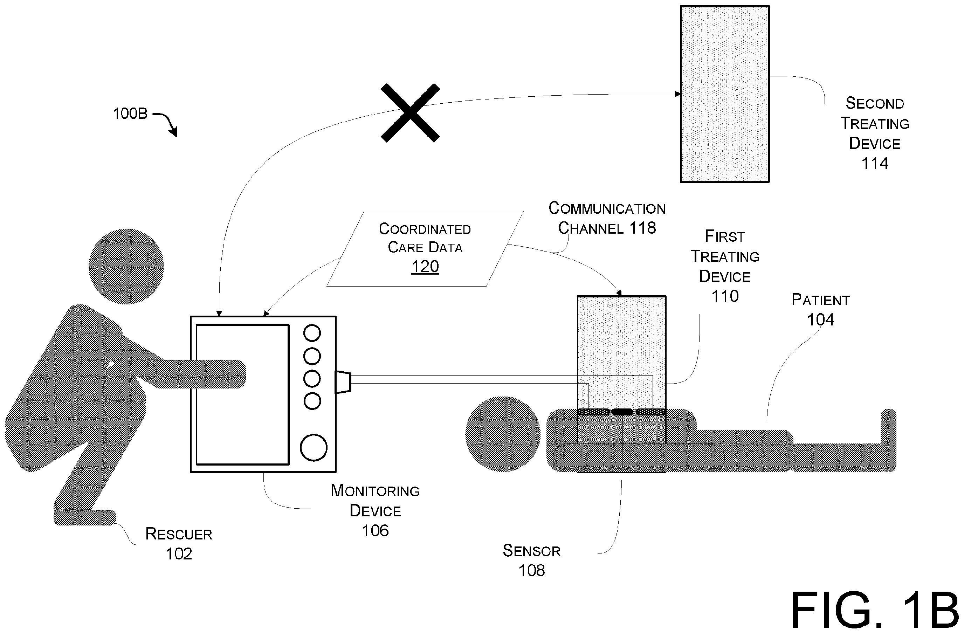

FIGS. 1A and 1B illustrate example environments for establishing communications between medical devices at a rescue scene. The rescue scene, in some implementations, is within a clinical environment, such as a hospital or other managed care facility. In some cases, the rescue scene is outside of a clinical environment.

FIG. 1A illustrates the rescue scene 100A at a first time. In various implementations, a rescuer 102 is treating a patient 104 at the rescue scene 100A. For instance, the rescuer 102 is an EMT professional monitoring and/or treating a medical condition of the patient 104. In some cases, the patient 104 is experiencing cardiac arrest or some other dangerous medical condition.

The rescuer 102 is monitoring the condition of the patient 104 using a monitoring device 106. For instance, the monitoring device 106 may be a monitor-defibrillator, a medical imaging device, an ultrasound monitor, a standalone ECG monitor, or another type of patient monitor. The monitoring device 106 includes and/or is communicatively coupled to a sensor 108. The sensor 108 is configured to detect at least one physiological parameter of the patient 104. Examples of physiological parameters include, for instance, an ECG, an impedance, a force administered to the patient 104, a blood pressure, an airway parameter (e.g., a partial pressure of carbon dioxide, a partial pressure of oxygen, a capnograph, an end tidal gas parameter, a flow rate, etc.), a blood oxygenation (e.g., a pulse oximetry value, a regional oximetry value, etc.), an electroencephalogram (EEG), a temperature, a heart sound, a blood flow rate, a physiological geometry (e.g., a shape of a blood vessel, an inner ear shape, etc.), a heart rate, a pulse rate, or the like. For example, the sensor 108 includes at least one of electrodes, a detection circuit, defibrillator pads, a force sensor, a blood pressure cuff, an ultrasound-based blood pressure sensor, an invasive (e.g., intra-arterial) blood pressure sensor (e.g., including a cannula inserted into the patient 104), a gas sensor (e.g., a carbon dioxide and/or oxygen sensor), a flowmeter, a pulse oximetry sensor a regional oximetry sensor, a thermometer, a microphone, an ultrasound transducer, a medical imaging device (e.g., an ultrasound imaging device), or the like. In various cases, the monitoring device 106 outputs the physiological parameter(s) to the rescuer 102. For instance, the monitoring device 106 includes a display, a speaker, or haptic feedback device that conveys the physiological parameter(s) to the rescuer 102.

A first treating device 110 (also referred to as a "treatment device") administers a treatment to the patient 104. For example, the first treating device 110 is a monitor-defibrillator, an automated external defibrillator (AED), mechanical chest compression device, a smart bag-valve mask, a ventilator, a heart-lung machine, an intravenous fluid (IV) pump, or the like. Examples of treatments include defibrillation, pacing, cardioversion, administration of chest compressions, administration of oxygen to the airway of the patient 104, movement of air in the airway of the patient, administration of fluids to the patient 104, extracorporeal membrane oxygenation (ECMO), administration of a medication to the patient 104, or the like. In some implementations, the monitoring device 106 is also configured to administer a treatment to the patient 104. Further, in some cases, the first treating device 110 is configured to detect one or more physiological parameters of the patient 104.

In some cases, the monitoring and/or treatment of the patient 104 is optimized by communication between the monitoring device 106 and the first treating device 110. In particular examples, the monitoring device 106 or the first treating device 110 reports a detected physiological parameter to the other device. In some cases, the monitoring device 106 or the first treating device 110 reports a treatment parameter to the other device. For instance, the first treating device 110 may include a blood pressure sensor and may report a blood pressure of the patient 104 to the monitoring device 106. In some examples, the first treating device 110 reports a frequency of chest compressions administered by the first treating device 110 to the monitoring device 106. The receiving device may perform one or more actions based on the physiological parameter and/or the treatment parameter. Actions performed by the monitoring device 106 or the first treating device 110 include initiating a measurement of a physiological parameter at a particular time or frequency, outputting a signal to the rescuer 102, outputting a signal to the patient 104, performing a treatment at a particular time or frequency, adjusting a treatment parameter of an ongoing treatment, or any combination thereof. According to some implementations, the monitoring device or the first treating device 110 instructs the other device to perform one or more actions. The receiving device, in turn, performs the action(s) based on the instruction from the monitoring device 106 or the first treating device 110. For instance, the monitoring device 106 instructs the first treating device 110 to cease administering chest compressions to the patient 104 at a predetermined time, and the monitoring device 106 administers a defibrillation shock to the patient 104 at the particular time. By exchanging reports, instructions, or other data, the monitoring device 106 and the first treating device 110 can coordinate monitoring and treatment of the patient 104.

To exchange data, the monitoring device 106 and/or the first treating device 110 are configured to establish and/or communicate via a communication channel. As used herein, the term "communication channel," and its equivalents, may refer to a medium over which a first endpoint (e.g., a sender) transmits information to one or more second endpoints (e.g., receivers). Examples of communication channels include wired connections, such as Ethernet or fiber optic paths, as well as wireless connections, such as Institute of Electronics and Electrical Engineers (IEEE) (e.g., WI-FI, BLUETOOTH, etc.) or 3^{rd} Generation Partnership Program (3GPP) (e.g., Long Term Evolution (LTE), New Radio (NR), etc.) connections. As used herein, the term "endpoint," and its equivalents, may refer to an entity that is configured to transmit and/or receive data. Examples of endpoints include user equipment (UE) (e.g., mobile phones, tablet computers, etc.), computers, base stations, access points (APs), servers, compute nodes, medical devices, Internet of Things (IoT) devices, and the like.

In some implementations, the communication channel between the monitoring device 106 and the first treating device 110 is established when the monitoring device 106 and the first treating device 110 are paired. In particular cases, the monitoring device 106 and first treating device 110 refrain from sharing substantive data (e.g., physiological metrics, reports about the patient 104, instructions for treating the patient 104, etc.) until the monitoring device 106 and the first treating device 110 are paired. As used herein, the term "paired," and its equivalents, may refer to a state of multiple devices that have a shared link key that enables each device to cryptographically authenticate data it receives from any other device among the multiple devices.

In particular cases, a first paired device encrypts data prior to transmitting the data to a second paired device, and the second paired device restores the original data by decrypting the encrypted data. As used herein, the term "encrypt," and its equivalents, refers to a process of translating data from one format (e.g., an unencoded format) into an encoded format. In various cases, the encoded format is referred to as "ciphertext." Unencoded data, which has not been encrypted, may be referred to as being in "plaintext." In various examples, an entity encrypts data using at least one encryption key. An encryption key is a parameter that defines the translation of data from the one format into the encoded format. As used herein, the term "decrypt," and its equivalents, refers to a process of translating data from an encoded format into another format (e.g., an unencoded format), such as a plaintext format. In various examples, an entity encrypts data using at least one decryption key. A decryption key is a parameter that defines the translation of data from the encoded format into the other format. A link key, for example, is an encryption and/or decryption key.

Various cryptographic techniques can be utilized in accordance with the features described in this disclosure. For example, data can be encrypted and decrypted via a symmetric key, wherein the encryption key and the decryption key are equivalent. In some cases, data can be encrypted and decrypted via asymmetric keys, wherein the encryption key and the decryption key are different. Cryptographic hash functions (CHFs) are examples of cryptographic techniques. Examples of cryptographic techniques include the Data Encryption Standard (DES), Advanced Encryption Standard (AES), Elliptic Curve Cryptography (ECC), Rivest-Shamir-Adleman (RSA), Secure Hash Algorithm (SHA)-1, SHA-2, SHA-3, BLAKE, BLAKE2, BLAKE3, WHIRLPOOL, MD2, MD4, MD5, MD6, Temporal Key Integrity Protocol (TKIP), Rivest cipher 4 (RC4), variably modified permutation composition (VMPC), blowfish, Twofish, Threefish, Tiny Encryption Algorithm (TEA), Extended TEA (XTEA), Corrected Block TEA (XXTEA), Diffie-Hellman exchange (DHE), elliptic curve DHE, supersingular isogeny Diffie-Hellman (SIDH) key exchange, and so on. Any suitable encryption or decryption technique can be used in accordance with implementations of this disclosure.

Various mechanisms can be utilized to pair the monitoring device 106 with the treating device 110. For example, automated pairing may involve the exchange of data and/or pairing requests/responses between the devices 106 and 110. As another example, the monitoring device 106 may receive an input signal from an operator (e.g., the rescuer 102) that selects the treating device 110 as a device to pair with the monitoring device 106, or vice versa. In some cases, the monitoring device 106 detects an alternative signal (e.g., a flashing light pattern) from the treating device 110 that is indicated in a pairing request. In some cases, the monitoring device 106 and the treating device 110 are paired, at least in part, based on signaling to and/or from an intermediary device (not illustrated in FIGS. 1A and 1B). In a particular example, two or more devices can be brought into proximity to (e.g., into contact with) each other in order to facilitating pairing the monitoring device 106 with the treating device 110. For example, a "tap-to-pair" functionality may allow a user to bring the monitoring device 106 (or a component thereof) into close proximity to (e.g., into contact with) the treating device 110 (or a component thereof), or vice versa, and a short-range wireless protocol, such as BLUETOOTH, near-field communication (NFC), or the like, may be used to detect that the devices 106 and 110 are within a threshold distance of each other, and, in response, the devices 106 and 110 may be paired. In some cases, an intermediary device, such as a phone, may be brought into close proximity to (e.g., into contact with) the monitoring device 106 and/or the treating device 110 (e.g., by touching the intermediary device to both devices 106 and 110 sequentially), and a short-range wireless protocol may be used to detect these proximity events involving the intermediary device, and, in response, the devices 106 and 110 may be paired.

In various cases, the monitoring device 106 and the first treating device 110 are paired when the first treating device 110 transmits a first pairing request 112 to the monitoring device 106 and the monitoring device 106 pairs with the first treating device 110 based on the first pairing request 112. The first pairing request 112, according to some cases, is part of a handshake between the monitoring device 106 and the first treating device 110. As used herein, the term "handshake," and its equivalents, refers to one or more signals transmitted between at least two endpoints that establish protocols of communication between the endpoints (e.g., prior to substantive data being exchanged between the endpoints). The monitoring device 106 and the first treating device 110, in some cases, utilize Transmission Control Protocol (TCP). TCP, for instance, utilizes handshakes to establish communication sessions between devices. For example, a first device transmits a synchronize message including a first sequence number to a second device. The second device, in turn, transmits a synchronize-acknowledgement message including a second sequence number as well as a first acknowledgement number that is one greater than the first sequence number. In response, the first device transmits an acknowledgement message including a second acknowledgment number that is one greater than the second sequence number to the second device. The first and second devices, in some examples, are paired once the first device transmits the acknowledgement message. The sequence and acknowledgement numbers are used to initialize counters that track messages and/or bytes of data transmitted between the paired devices.

In some examples, the first pairing request 112 includes or otherwise indicates a link key that enables the monitoring device 106 to pair with the first treating device 110. The first treating device 110, for instance, broadcasts the first pairing request 112 into the rescue scene 100. In some examples, the first treating device 110 broadcasts the first pairing request 112 in response to receiving an input signal from the rescuer 102 (e.g., the rescuer has pushed a button on the first treating device 110), in response to being powered on, in response to initiating the treatment of the patient 104, or the like.

However, as illustrated in FIG. 1A, a second treating device 114 is also in the rescue scene 100A. The second treating device 114 transmits a second pairing request 116 to the monitoring device 106. Thus, the monitoring device 106 receives the first pairing request 112 and the second pairing request 116. Unlike the first treating device 110, the second treating device 114 is not administering a treatment patient 104 or monitoring a condition of the patient 104. In some implementations, the second treating device 114 is administering a treatment to a different individual at the rescue scene 100A. If the monitoring device 106 pairs to the second treating device 114, rather than the first treating device 110, the care of the patient 104 and/or the different individual may be compromised.

For instance, in a particular case, the monitoring device 106 receives an instruction to administer a defibrillation shock to the patient 104. The first treating device 110 is administering chest compressions to the patient 104. The monitoring device 106 may transmit an instruction to cease administering the chest compressions prior to administering the defibrillation shock. However, if the monitoring device 106 transmits the instruction to the second treating device 114, rather than the first treating device 110, the first treating device 110 will not cease administering the chest compressions before the defibrillation shock is to be administered, which could potentially delay administration of the defibrillation therapy to the patient 104. Furthermore, if the second treating device 114 is administering chest compressions to a different individual who is in need of chest compressions, and the second treating device 114 ceases administering the chest compressions, then the different individual may experience dangerous levels of hypoxemia.

Various implementations of the present disclosure relate to pairing the correct medical devices. For instance, techniques described herein enable the monitoring device 106 to pair with the first treating device 110, which is administering the treatment to the patient 104, rather than the second treating device 114, which is not administering a treatment to the patient 104.

In some examples, the monitoring device 106 distinguishes between the first pairing request 112 and the second pairing request 116 based on a signal detected by the sensor 108. In various cases, the signal is indicative of the treatment administered by the first treating device 110. According to various implementations, the signal detected by the sensor 108 is referred to as a "parameter signal." The monitoring device 106, in some cases, detects the treatment by analyzing the parameter signal. For instance, the monitoring device 106 identifies the treatment based on a time, frequency, or shape of an artifact in the parameter signal that corresponds to the treatment. For instance, a chest compression artifact in an ECG can be recognized as a periodic spike in the ECG at a frequency consistent with chest compression administration. In various implementations, the monitoring device 106 identifies the type of device administering the treatment based on the artifact. For example, a chest compression artifact produced one type of mechanical chest compression device is shaped differently than a chest compression artifact produced by another type of mechanical chest compression device. The monitoring device 106, in some cases, determines a parameter of the treatment by analyzing the signal detected by the sensor 108. This type of parameter, as described herein, can be referred to as a "treatment parameter." Examples of treatment parameters include timing, frequency, shape, or magnitude of the treatment.

Further, the first pairing request 112 indicates the treatment administered by the first treating device 110. In some implementations, the first pairing request 112 includes the treatment parameter. According to some cases, the monitoring device 106 derives the treatment parameter based on the first pairing request 112. In various cases, the first pairing request 112 includes a frequency of the treatment, a timing of the treatment, a magnitude of the treatment, a model of the first treating device 110 administering the treatment, or the like. In some cases, the second pairing request 116 indicates the treatment administered by the second treating device 114. The monitoring device 106 can therefore determine that the first pairing request 112 is from the first treating device 110, which is administering the treatment to the patient 104, based on the signal detected by the sensor 108.

In particular implementations, the monitoring device 106 is a monitor-defibrillator and the first treating device 110 is a mechanical chest compression device. The sensor 108, for example, includes an ECG sensor (e.g., multiple electrodes disposed on the chest of the patient 104) that is configured to detect a signal that includes the ECG of the patient 104. In various implementations, the signal detected by the sensor 108 also includes an artifact that is present due to chest compressions being administered to the patient 104 by the first treating device 110. The monitoring device 106 is configured to analyze the signal detected by the ECG sensor. In various cases, the monitoring device 106 identifies the timing and/or frequency of the chest compressions administered to the patient 104. The first pairing request 112 may indicate the timing and/or frequency of the chest compressions being administered by the first treating device 110. The monitoring device 106 may therefore confirm that the first pairing request 112 is received from the device treating the patient 104 by comparing the timing and/or frequency of the chest compressions identified in the signal detected by the sensor 108 with the indication(s) in the first pairing request 112. Further, a timing and/or frequency of chest compressions indicated by the second treating device 114 in the second pairing request 116 may be inconsistent with the chest compressions detected by the sensor 108, such that the monitoring device 106 may determine that the second pairing request 116 is not received by the device treating the patient 104. Therefore, the monitoring device 106 may pair to the first treating device 110 based on the key indicated by the first pairing request 112, and may refrain from pairing to the second treating device 114 based on the key indicated in the second pairing request 116.

Other mechanisms are optionally utilized to pair the monitoring device 106 with the first treating device 110. For instance, the monitoring device 106 may receive an input signal from the rescuer 102 that selects the first treating device 110 rather than the second treating device 114. In some cases, the monitoring device 106 detects an alternative signal (e.g., a flashing light pattern) from the first treating device 110 that is indicated in the first pairing request 112. In some cases, the monitoring device 106 and the first treating device 110 are paired, at least in part, based on signaling to and/or from an intermediary device (not illustrated).

FIG 1B illustrates the rescue scene 100B at a second time. In various implementations, the monitoring device 106 pairs with the first treating device 110 between the first and second time. Because the monitoring device 106 and the first treating device 110 are paired, the monitoring device 106 and the first treating device 110 establish a communication channel 118 and exchange coordinated care data 120 over the communication channel 118. In contrast, the monitoring device 106 has refrained from establishing a communication channel with the second treating device 114.

The coordinated care data 120 includes substantive data relevant to monitoring and/or treating the patient 104. In some implementations, the coordinated care data 120 indicates one or more physiological parameters of the patient 104. For example, the first treating device 110 may detect a physiological parameter of the patient 104 and report the physiological parameter to the monitoring device 106. In some implementations, the monitoring device 106 outputs the physiological parameter to the rescuer 102, such as on a display of the monitoring device 106. In some cases, the monitoring device 106 determines a condition of the patient 104 based on the physiological parameter detected by the first treating device 110. For instance, first treating device 110 may detect and report a blood pressure of the patient 104 to the monitoring device 106 in the coordinated care data 120, and the monitoring device 106 may determine whether the patient 104 has return of spontaneous circulation (ROSC) by analyzing the blood pressure and the ECG of the patient 104.

In various cases, the coordinated care data 120 includes one or more instructions. The monitoring device 106, for instance, instructs the first treating device 110 to detect a physiological parameter of the patient 104, to administer a treatment to the patient 104, to cease administering a treatment to the patient 104, to modify a treatment of the patient 104, or any combination thereof. In some cases, the first treating device 110 instructs the monitoring device 106 to detect a physiological parameter of the patient, to administer a treatment to the patient 104, to cease administering a treatment to the patient 104, to modify a treatment of the patient 104, or any combination thereof.

According to some examples, the coordinated care data 120 includes synchronization data that enables the monitoring device 106 and the first treating device 110 to synchronize clocks. As used herein, the term "clock," and its equivalents, may refer to hardware and/or software of a device that tracks time. In various examples, the monitoring device 106 includes a first clock and the first treating device 110 has a second clock. Before the monitoring device 106 and the first treating device 110 are paired, their clocks may be unsynchronized, such that the devices may perceive a particular time at different actual times. In some implementations, the coordinated care data 120 includes data that enables the monitoring device 106 and the first treating device 110 to be able to perceive a particular time at the same actual time. For example, if the monitoring device 106 instructs the first treating device 110 to administer a chest compression at a particular time in the future, the synchronized clocks enable the first treating device 110 to actually administer the chest compression at the particular time.

In some implementations, the synchronization data includes a token, which includes data indicating a time stamp generated by the sender based on its own time domain (e.g., as indicated by its own clock). The receiver of the synchronization data may register the time stamp within its own time domain (e.g., as indicated by its own clock), such that the receiving device can identify any time delay between the clock of the sender and the clock of the receiver. The receiver, for instance, may use the time delay to translate reports and/or instructions that are associated with a particular time specified by the sender.

A particular example will now be described with reference to FIGS. 1A and 1B, wherein the monitoring device 106 is a monitor-defibrillator and the first treating device 110 and the second treating device 114 are mechanical chest compression devices. The sensor 108, for example, includes ECG electrodes affixed to the chest of the patient 104. The monitoring device 106 includes a detection circuit configured to detect a voltage between the ECG electrodes over time. The voltage over time, for instance, includes the ECG of the patient 104. The first treating device 110 is administering chest compressions to the patient 104. Due to the chest compressions, the voltage between the ECG electrodes over time also includes an artifact. The monitoring device 106 determines that the chest compressions are applied to the patient 104 by identifying the artifact in the voltage overtime. In addition, the monitoring device 106 determines the frequency at which the chest compressions are administered by determining the frequency at which the artifact appears in the voltage over time. The first pairing request 112, transmitted to the monitoring device 106 by the first treating device 110, indicates the frequency of the chest compressions. Meanwhile, the second pairing request 116, transmitted to the monitoring device 106 by the second treating device 114, indicates a different frequency or indicates that the second treating device 114 is not currently administering compressions. By determining that the voltage detected by the sensor 108 and the first pairing request 112 indicate the same chest compression frequency, the monitoring device 106 automatically pairs with the first treating device 110 based on the first pairing request 112. Once paired, the monitoring device 106 and the first treating device 110 communicate over a wireless communication interface, such as a BLUETOOTH interface.

Once paired, the monitoring device 106 and the first treating device 110 coordinate monitoring and treatment of the patient 104. For instance, the first treating device 110 transmits a message reporting that the first treating device 110 is changing the frequency at which the chest compressions are administered to the patient 104. The first treating device 110 identifies, selects, or generates a digital filter based on the new chest compression frequency. For instance, the monitoring device 106 generates a comb filter that rejects a band corresponding to the new chest compression frequency as well as one or more harmonics of the new chest compression frequency. The monitoring device 106 removes the chest compression artifact by applying the digital filter to the voltage over time detected by the sensor 108. The patient's 104 ECG is more pronounced in the resultant signal than the signal in which the chest compression artifact is present.

In this particular example, the monitoring device 106 determines that the patient 104 is experiencing ventricular fibrillation (VF) by analyzing the ECG of the patient 104. VF is treatable by defibrillation. However, in some cases, the patient 104 may be harmed if the monitoring device 106 administers an electrical shock while the first treating device 110 is administering chest compressions. To avoid the potential for injury to the patient 104, the monitoring device 106 transmits, to the first treating device 110, an instruction to pause the chest compressions. Once the chest compressions are paused, the monitoring device 106 outputs an electrical shock to the heart of the patient 104. In some cases, the first treating device 110 begins administering chest compressions to the patient 104 immediately after the patient 104 receives the electrical shock. For instance, the instruction specifies a length of the chest compression pause, or the monitoring device 106 transmits another instruction to resume chest compressions after the electrical shock has been discharged.

In another particular example, the monitoring device 106 is a patient monitor and the first treating device 110 is an ECMO or other type of extra corporeal life support (ECLS) device that removes carbon dioxide from the blood of the patient 104, increases oxygen in the blood of the patient 104, and pumps the blood through the body of the patient 104. The monitoring device 106 determines that the patient 104 is receiving an ECMO treatment by determining that a variance of an invasive blood pressure (e.g., arterial blood pressure) of the patient 104 over time is below threshold, which indicates that the blood flowing through the patient 104 is non-pulsatile. In various cases, the first pairing request 112 indicates a shape of the blood pressure waveform implemented by the first treating device 110, such that the monitoring device 106 confirms that the first pairing request 112 is received from the device treating the patient 104. Once the devices are paired, in some instances, the monitoring device 106 determines that a peripheral oxygenation of the patient 104 is below a threshold. In response, the monitoring device 106 transmits an instruction to increase the oxygenation rate of the first treating device 110 (or to otherwise adjust the sweep of the device).

In a further example, the monitoring device 106 is an ultrasound monitor and the first treating device 110 is a ventilation device configured to introduce oxygen to the airway of the patient 104 at a particular respiration rate. An ultrasound transducer of the monitoring device 106 is configured to emit ultrasound into the patient 104 (e.g., at a neck of the patient 104) and to detect ultrasound scattered from the patient 104. The monitoring device 106 generates an image of a portion of the patient 104 by analyzing the emitted and detected ultrasound. In some cases, the monitoring device 106 determines that the patient 104 is receiving a ventilation treatment by detecting periodic movement of the portion of the patient 104, which could be detected using speckle tracking. In this example, the first pairing request 112 indicates a ventilation rate administered by the first treating device 110. If the frequency of movement in the ultrasound image is consistent with the ventilation rate indicated by the first treating device 110, the monitoring device 106 initiates pairing with the first treating device 110. Once paired, the monitoring device 106 detects that the patient 104 is experiencing gastric insufflation. In response, the monitoring device 106 transmits an instruction to the first treating device 110 that causes the first treating device 110 to lower a flow rate of air (including oxygen) administered to the patient 104.

Although the monitoring device 106 is illustrated in FIGS. 1A and 1B as a monitor-defibrillator, implementations are not so limited. For instance, the monitoring device 106 may be a mechanical chest compression device, an imaging device, or any other device configured to detect a parameter of the patient 104. In addition, the treating device 110 could be a monitor-defibrillator, a mechanical chest compression device, or any other device configured to administer a treatment (e.g., an electrical shock, pacing, chest compressions, etc.) to the patient 104.

In FIGS. 1A and 1B, the monitoring device 106 and first treating device 110 are illustrated as communicating directly. However, implementations are not so limited. For example any illustrated and/or described communication between the monitoring device 106 and the first treating device 110 can be relayed by at least one intermediary device.

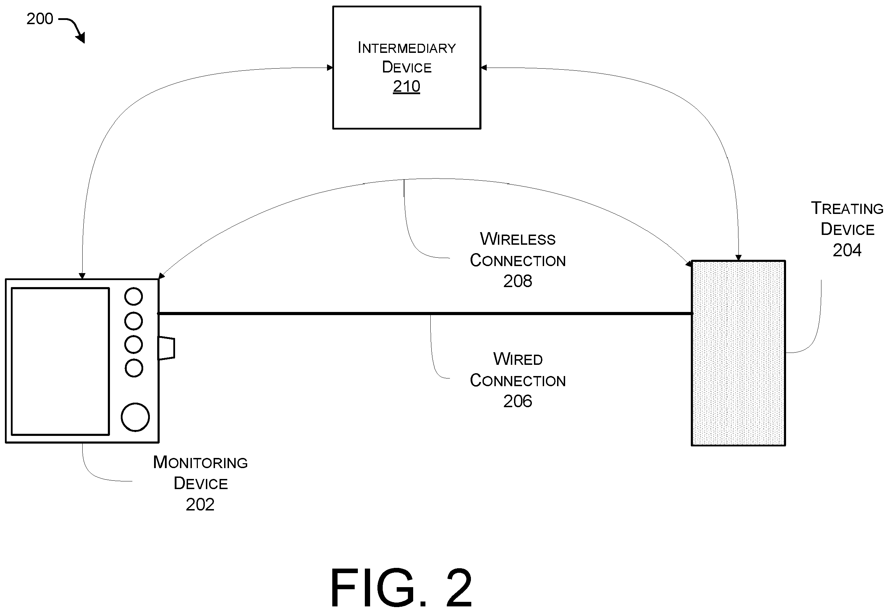

FIG. 2 illustrates an example environment 200 in which a monitoring device 202 and a treating device 204 are configured to communicate via one or more connections. In some examples, the monitoring device 202 is the monitoring device 106 described above with reference to FIGS. 1A and 1B, and the treating device 104 is the first treating device 110 or the second treating device 114 described above with reference to FIGS. 1A and 1B.

In some implementations, the monitoring device 202 and the treating device 204 are configured to exchange data over a wired connection 206. In some cases, the wired connection 206 is a physical cable connected to the monitoring device 202 and the treating device 204. The wired connection 206, for instance, is removably coupled to the monitoring device 202 and/or the treating device 204. For instance, a user may physically couple the wired connection 206 to a socket of the monitoring device 202 and/or a socket of the treating device 204. In various cases, data is transmitted over the wired connection 206 via electrical and/or optical signals. Examples of sockets include Universal Service Bus (USB) sockets, micro-USB sockets, sockets specific to medical connectors, and the like.

According to some implementations, the wired connection 206 supports reliable and low-latency communication between the monitoring device 202 and the treating device 204, but may physically impede movements of a rescuer operating the monitoring device 202 and/or treating device 204, movements of a patient being monitored and/or treated by the monitoring device 202 and/or the treating device 204, or even movements of the monitoring device 202 and/or the treating device 204 themselves. In some cases, the wired connection 206 connects the monitoring device 202 and the treating device 204 temporarily during the treatment of a patient at a rescue scene. For instance, the monitoring device 202 and the treating device 204 may exchange data that enables the monitoring device 202 and the treating device 204 to pair and exchange substantive data over an alternate communication channel.

In some cases, the monitoring device 202 and the treating device 204 are configured to exchange data over a wireless connection 208. Examples of the wireless connection 208 include an ultrasonic connection, a Li-Fi connection, an IEEE connection (e.g., a WI-FI, BLUETOOTH, ZIGBEE, etc.), a 3GPP connection (e.g., LTE, NR, etc.), a Global System for Mobile Communications Association (GSMA) connection, a European Telecommunications Standards Institute (ETSI) connection, or the like. In various instances, data is transmitted over the wireless connection 208 via electromagnetic (e.g., RF signaling, visible light, etc.) and/or ultrasonic signals. In some examples, one or more base stations and/or APs facilitate the wireless connection 208 between the monitoring device 202 and the treating device 204.

In various cases, data transmitted over the wireless connection 208 is encrypted. For example, the monitoring device 202 encrypts data, the monitoring device 202 transmits the encrypted data over the wireless connection 208 (e.g., as a wireless signal), and the treating device 204 restores the data by decrypting the encrypted data. Similarly, in some cases, the treating device 204 encrypts data, the treating device 204 transmits the encrypted data over the wireless connection 208 (e.g., as a wireless signal), and the monitoring device 202 restores the data by decrypting the encrypted data. In some cases, the encrypted data includes substantive data, such as physiological parameters, reports, and instructions. In some implementations, the monitoring device 202 and treating device 204 perform encryption and/or decryption using one or more keys. The key(s), for instance, is exchanged during pairing.

According to various implementations, the monitoring device 202 and the treating device 204 are configured to exchange data via an intermediary device 210. The intermediary device 210, for example, includes a passkey, a badge, a card, a mobile device, a tablet computer, an loT device, a computing device, a server, a hub device, a smart cot device, another medical device, or any combination thereof. As used herein, the term "passkey," and its equivalents, may refer to a passive device including an antenna that is powered by an electromagnetic field generated by a separate, active device. That is, a passkey may be powered by inductive coupling. In some cases, when powered, the passkey may act as a transponder by emitting an electromagnetic signal that can be detected by the active device. For instance, the passkey may be a passive NFC target device. In some implementations, the passkey is an ID badge of a rescuer operating the monitoring device 202 and/or the treating device 204.

In some examples, the intermediary device 210 includes or is integrated into a cot. As used herein, the term "cot," and its equivalents, may refer to a portable patient support apparatus. A cot, for instance, may be utilized by one or more rescuers to move a patient from one location to another location. In particular examples, the cot includes a cushioned bed surface that supports the patient in a supine position. The cot, in addition, may include an adjustable frame configured to change and/or support an angle or height at which the bed is disposed. In some cases, the cot further includes retention straps configured to bind the patient to the bed. In some cases, the frame is mechanically coupled to a support that is configured to hold the monitoring device 202 and/or the treating device 204. In addition, the cot may include one or more processors and/or transceivers configured to perform various functions described herein.

In some cases, the monitoring device 210 includes or is integrated into a hub device. As used herein, the terms "hub," and "hub device," and their equivalents, may refer to a device configured to connect and exchange data with multiple external devices. For instance, a hub device may act as an AP or base station connected to multiple devices. The hub device, for example, is portable. In some cases, the hub device is integrated with a transportation device, such as an ambulance, that is configured to move between different locations. The hub device, for instance, may include one or more processors and/or transceivers configured to perform various functions described herein.

The intermediary device 210 may be connected to the monitoring device 202 by a first wired and/or wireless connection, and may be connected to the treating device 204 by a second wired and/or wireless connection. In some implementations, at least one wireless connection that connects the intermediary device 210 to the monitoring device 202, or the intermediary device 210 to the treating device 204, is of a different protocol than the wireless connection 208. For example, the intermediary device 210 may exchange data with the monitoring device 202 and/or the treating device 204 via NFC signaling, whereas data transmitted over the wireless connection 208 may be in the form of RF signaling (e.g., BLUETOOTH signaling).

In various implementations, the intermediary device 210 facilitates pairing between the monitoring device 202 and the treating device 204. For instance, once paired, the monitoring device 202 and the treating device 204 establish and/or exchange data over the wireless connection 208. Further, once paired, the monitoring device or the treating device 204 may rely on parameters reported by the other device, or may perform actions instructed by the other device.

In some implementations, the intermediary device 210 transmits a key to the monitoring device 202 and/or the treating device 204. The monitoring device 202 and/or the treating device 204 use the key to encrypt and/or decrypt data transmitted over the wireless connection 208. In some cases, the intermediary device 210 receives the key from the monitoring device 202 and/or the treating device 204. According to some examples, the intermediary device 210 generates the key. In some examples, the intermediary device 210 provides multiple keys to the monitoring device 202 and/or the treating device 204 for ongoing communication between the monitoring device 202 and the treating device 204.

According to some implementations, the intermediary device 210 pairs with the monitoring device 202 and/or the treating device 204. For instance, the intermediary device 210 may receive a signal from the monitoring device 202 that advertises an identity of the monitoring device 202 (e.g., a numerical identifier associated with the monitoring device 202). A user may operate the intermediary device 210 (for instance, a mobile phone) to select the monitoring device 202 from a drop-down menu output on a touchscreen of the intermediary device 210. In response to the selection, the monitoring device 202 and the intermediary device 210 pair. In some cases, the intermediary device 210 further receives a signal indicating an identity of the treating device 202. The user, for instance, operates the intermediary device 210 to select the treating device 204. In various implementations, the intermediary device 210 transmits data to the monitoring device 202 and/or the treating device 204 that causes pairing between the monitoring device 202 and the treating device 204.

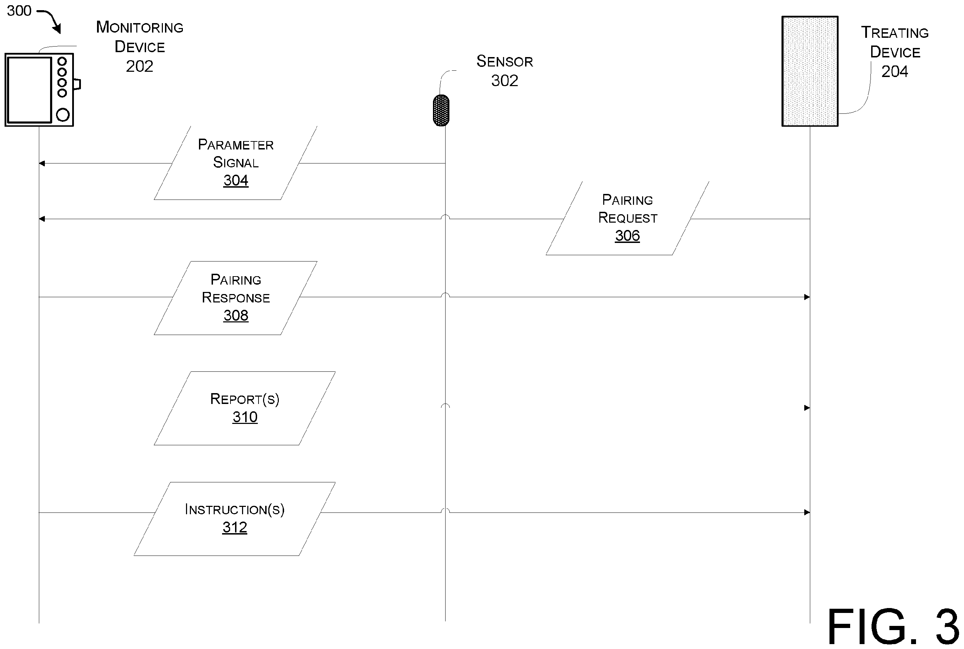

FIG. 3 illustrates example signaling 300 for facilitating communication between medical devices. In particular, the signaling 300 is between the monitoring device 202 and treating device 204 described above with reference to FIG. 2. Various messages within the signaling 300 are transmitted over at least one wired connection (e.g., wired connection 206) and/or at least one wireless connection (e.g., the wireless connection 208). Although not specifically illustrated, various messages within the signaling 300 are transmitted via one or more intermediary devices (e.g., the intermediary device 210).

The monitoring device 202 receives a parameter signal 304 from a sensor 302. The parameter signal 304 indicates one or more physiological parameters of an individual, such as a patient. For example, the parameter signal 304 includes at least one of an ECG, a transthoracic impedance, an airway parameter (e.g., a flow rate, a partial pressure of oxygen, a partial pressure of carbon dioxide, a capnograph, and end tidal parameter, etc.), a blood pressure, a blood oxygenation (e.g., regional oximetry, pulse oximetry, etc.), or the like. In various cases, the sensor 302 includes at least one of an electrode, a detection circuit, a flow sensor, an oxygen sensor, a carbon dioxide sensor, a non-invasive blood pressure (NIBP) sensor (e.g., a blood pressure cuff, an ultrasound-based blood pressure sensor, etc.), an oxygenation sensor (e.g., a regional oximetry sensor, a pulse oximetry sensor), or the like. In some implementations the monitoring device 202 includes the sensor 302.

The parameter signal 304, according to various examples, further indicates a treatment being performed on the individual by the treating device 204. For example, the parameter signal 304 indicates at least one of chest compressions, a ventilation treatment, a defibrillation treatment, a pacing treatment, or a medication being administered by the treating device 204 to the individual. In various cases, the treatment is indicated by an artifact present in the parameter signal 304. For instance, if the parameter signal 304 is an ECG, a chest compression artifact in the ECG indicates the administration of chest compressions to the individual. In various implementations, the monitoring device 202 identifies the treatment by analyzing the parameter signal 304. For instance, the monitoring device 202 determines a frequency and/or timing of the treatment by analyzing the parameter signal 304.

The monitoring device 202 also receives a pairing request 306 from the treating device 204. In some implementations, the pairing request 306 is broadcast by the treating device 204. The pairing request 306 includes pairing information. For instance, the pairing request 306 includes information that enables the monitoring device 202 to pair with the treating device 204. In some implementations, the pairing request 306 includes at least one of a key (an encryption and/or decryption key), an identity of the treating device 204, a communication capability of the treating device 204 (e.g., a channel over which the treating device 204 receives and/or transmits data wirelessly), or the like. Further, the pairing request 306 indicates the treatment administered by the treating device 204. For example, the pairing request 306 reports the frequency and/or timing of the treatment.

In various cases, the monitoring device 202 confirms that the treating device 204 is treating the individual that the monitoring device 202 is monitoring. For example, the monitoring device 202 determines that the treatment indicated by the parameter signal 304 is consistent with the treatment indicted by the pairing request 306.

The monitoring device 202 transmits a pairing response 308 to the treating device 204. In various implementations, the pairing response 308 includes information that enables the treating device 204 to pair with the monitoring device 202. For example, the pairing response 308 includes at least one of a key (an encryption and/or decryption key), an identity of the treating device 204, a communication capability of the treating device 204 (e.g., a channel over which the treating device 204 receives and/or transmits data wirelessly), or the like. In some cases, the pairing response 308 confirms that the monitoring device 202 will communicate via a communication channel proposed in the pairing request 306.

In some cases, the pairing request 306 and/or the pairing response 308 includes synchronization information. The synchronization information enables the monitoring device 202 and the treating device 204 to align their respective clocks. In some implementations, the pairing request 306 and/or the pairing response 308 includes at least one clock indication message. For instance, the treating device 204 may adjust an internal clock of the treating device 204 based on the pairing request 306 and/or the monitoring device 202 may adjust an internal clock of the monitoring device 202 based on the pairing response 308. A sending device may include a clock indication message that references a value of the clock of the sending device. In some implementations, the clock indication message is and/or includes a frequency hopping synchronization message.

Once paired, the monitoring device 202 and the treating device 204 exchange substantive data. In various implementations, the monitoring device 202 and/or the treating device 204 exchange one or more reports 310. For instance, the report(s) 310 include at least one physiological parameter, at least one treatment parameter, or a combination thereof.

In some cases, the monitoring device and/or the treating device 204 exchange one or more instructions 312. In some cases, the instruction(s) 312 include at least one instruction to measure a physiological parameter, to begin a treatment, to end a treatment, a particular time or frequency at which to perform an action, an instruction to power on, an instruction to power off, an instruction to disconnect from a patient being treated or monitored, or a combination thereof.

Data is transmitted between the monitoring device 202 and the treating device 204 asynchronously and/or synchronously. In some implementations, the monitoring device 202 and/or the treating device 204 transmits an example report among the report(s) 310 in response to an event. For example, the monitoring device 202 transmits a report indicating a physiological parameter of the individual in response to determining that the physiological parameter is outside of a predetermined range (e.g., the parameter is greater than a first threshold or lower than a second threshold). In some instances, the treating device 204 transmits an instruction to measure a physiological parameter in response to determining that the treatment is complete. For instance, the treating device 204 instructs the monitoring device 202 to detect an ECG of the individual in response to the treating device 204 pausing chest compressions. In some implementations, the treating device 204 periodically transmits reports indicating an ongoing treatment of the individual, such as every ten seconds. In some cases, the monitoring device 202 periodically transmits instructions to adjust the treatment based on real-time conditions of the monitored individual.

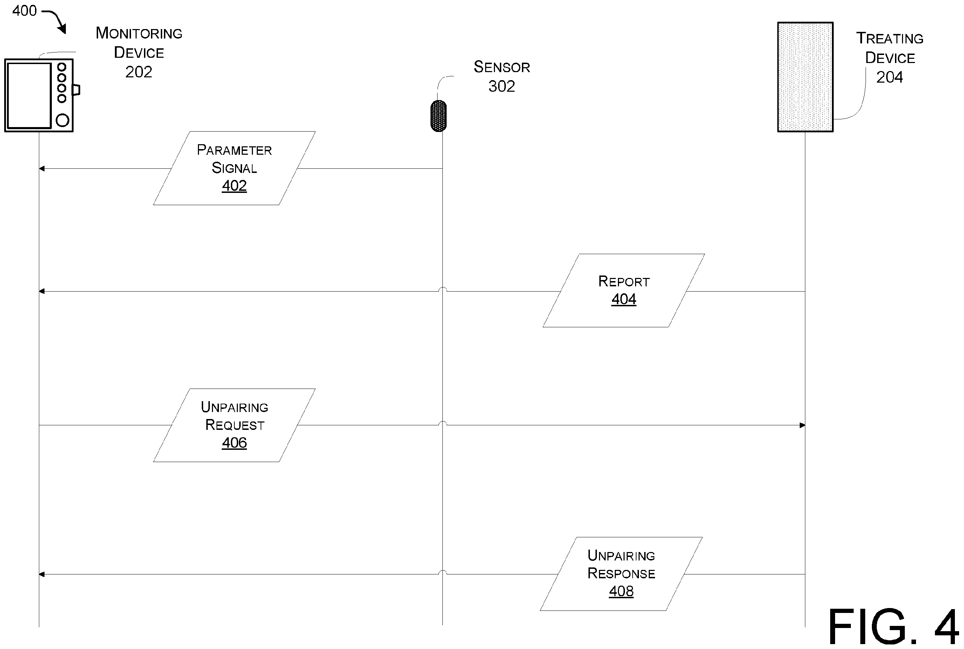

FIG. 4 illustrates example signaling 400 for ceasing communication between medical devices. As shown, the signaling 400 is between the monitoring device 202, treating device 204, and sensor 302 described above with reference to FIGS. 2 and 3.

In various implementations, the monitoring device 202 and the treating device 204 are paired. The monitoring device 202 has monitored an individual (e.g., a patient) that has also been treated by the treating device 204. However, the treating device 204 may no longer be treating the individual. For example, the treating device 204 may be disconnected from the individual. The signaling 400 can be performed to unpair the monitoring device 202 and the treating device 204 when the treating device 204 is no longer treating the individual.

The monitoring device 202 receives a parameter signal 402 from the sensor 302. The parameter signal 402 indicates one or more physiological parameters of an individual, such as a patient. For example, the parameter signal 402 includes at least one of an ECG, a transthoracic impedance, an airway parameter (e.g., a flow rate, a partial pressure of oxygen, a partial pressure of carbon dioxide, a capnograph, and end tidal parameter, etc.), a blood pressure, a blood oxygenation (e.g., regional oximetry, pulse oximetry, etc.), or the like.

In various cases, the parameter signal 402 lacks an indication of a treatment. For example, if the treating device 204 was administering a treatment to the individual, an artifact would be present in the parameter signal 402 indicating the treatment. However, if the artifact is absent from the parameter signal 402, the monitoring device 202 determines that the parameter signal 402 lacks the indication of the treatment.

Optionally, the monitoring device 202 receives a report 404 from the treating device 204. In some cases, the report 404 indicates a treatment being administered by the treating device 204. In some cases, the report 404 includes a treatment parameter characterizing the treatment. For instance, if the treating device 204 is a chest compression device, the report 404 indicates that the treating device 204 is administering compressions. In some cases, the report 404 indicates a physiological parameter detected by the treating device 204 (or lack thereof). For instance, the treating device 204 is configured to detect a blood pressure, but because the treating device 204 is removed from the individual, the report 404 may indicate a blood pressure of 0. In some cases, the treating device 204 transmits the report 404 in response to being commanded to power off or disconnect from the individual (e.g., by a user).

The monitoring device 202 determines that the treating device 204 is no longer associated with the individual based on the parameter signal 402 and/or the report 404. In some implementations, the monitoring device 202 determines that the parameter signal 402 no longer indicates a treatment administered by the treating device 204. However, in some cases, the monitoring device 202 may refrain from unpairing the treating device 204 based solely on a pause in the treatment administered by the treating device 204, since the treating device 204 may temporarily pause the treatment before restarting the treatment. For instance, the treating device 204 could perform a rhythm check that pauses chest compressions administered to the individual, but maintaining pairing between the monitoring device 202 and the treating device 204 may be preferred during the rhythm check. Optionally, the monitoring device 202 determines that a treatment administered by the treating device 204, as indicated in the report, is not reflected in the parameter signal 402. For instance, the monitoring device 202 determines that the parameter signal 202 lacks a chest compression artifact, but the report 404 indicates that the treating device 204 is administering chest compressions. In some cases, the monitoring device 202 determines that the parameter signal 202 indicates a treatment, but that the treatment indicated in the parameter signal 202 is different than the treatment indicated in the report 404. For instance, a chest compression artifact in the parameter signal 402 may have a first compression frequency, the chest compressions indicated in the report 404 may have a second compression frequency, wherein the first compression frequency is different than the second compression frequency. In some implementations, the monitoring device 202 determines that the treating device 204 is no longer associated with the individual based on receiving the report 404.

In response to determining that the treating device 204 is no longer associated with the individual, the monitoring device 406 refrains from transmitting reports and/or instructions to the treating device 204. In addition, the monitoring device 406 refrains from relying on and/or following reports and/or instructions received from the treating device 204.

In some implementations, the monitoring device 202 formally unpairs from the treating device 204 by transmitting an unpairing request 406 to the treating device 204. The unpairing request 406 may instruct the treating device 204 to refrain from communicating with the monitoring device 202. In some cases, the treating device 204 transmits an unpairing response 408 to the monitoring device 202. The unpairing response 408, for instance, confirms that the treating device 204 will refrain from transmitting and/or receiving data from the monitoring device 202 (unless they are paired together at a later time).

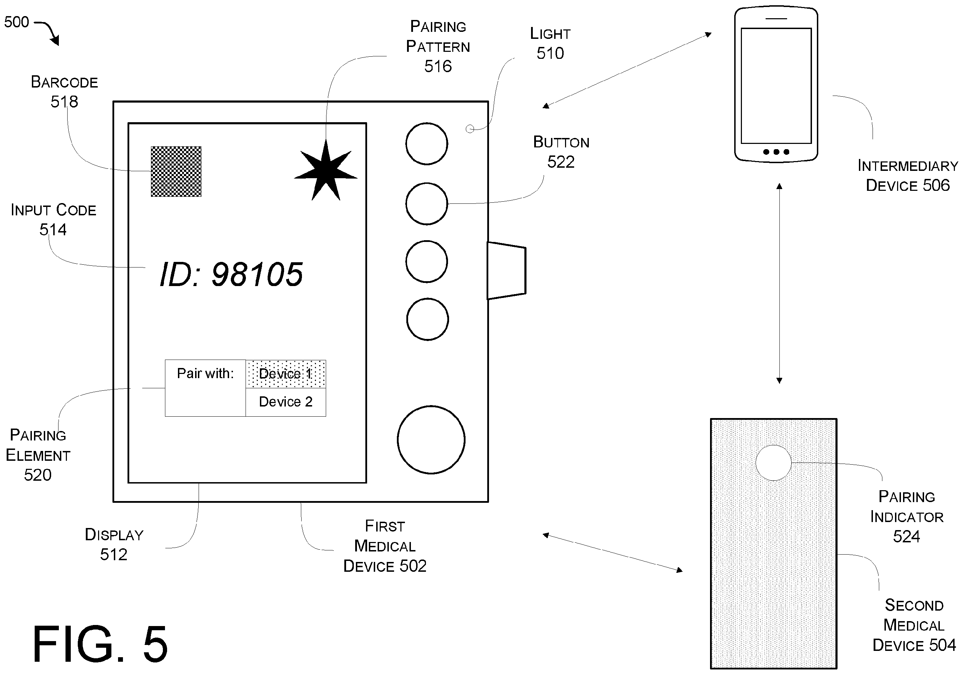

FIG. 5 illustrates an example environment 500 of interconnected devices that facilitate medical device pairing. In particular, the environment 500 includes a first medical device 502, a second medical device 504, and an intermediary device 506. The first medical device 502 and second medical device 504 may each be any medical device configured to monitor and/or treat an individual. In some cases, the intermediary device 506 is a third medical device (e.g., a smart cot) or a non-medical device, such as a general purpose computer, mobile device (e.g., mobile phone), tablet computer, loT device, hub, base station, AP, or the like.

The first medical device 502 includes one or more output devices configured to output signals to the second medical device 504 and/or the intermediary device 506. Although not specifically illustrated, the first medical device 502, optionally includes one or more transceivers configured to output signals to the second medical device 504 and/or the intermediary device 506. The output devices illustrated in FIG. 5 include a light 508 and a display 510.

The light 508 emits visible electromagnetic signals at a particular frequency. In some examples, the light 508 emits different colors of electromagnetic signals. According to some cases, the light 508 emits flashes of electromagnetic signals in a time-dependent pattern. In various implementations, the second medical device 504 and/or the intermediary device 506 detect the electromagnetic signals output by the light 508 and initiate pairing to the first medical device 502 based on the electromagnetic signals. For instance, the electromagnetic signals output by the light 510 indicates a channel and/or key associated with wireless communication with the first medical device 502. Upon detecting the electromagnetic signals output by the light 510, the second medical device 504 or intermediary device 506 initiates pairing with the first medical device 502 over the channel using the key as a link key. In some examples, the light 510 emits electromagnetic signals indicating that the first medical device 502 has paired with the second medical device 504 or the intermediary device 506, which can be perceived by a user. According to some instances, the light 510 indicates that the first medical device 502 is outputting a beacon wirelessly into the environment 500. As used herein, the term "beacon," and its equivalents, may refer to a signal that indicates a proximity, location, or pairing readiness of a device sending the beacon. Pairing requests, as described herein, are examples of beacons.

The display 512 is configured to visually output various signals that facilitate pairing. In some cases, the display 512 includes at least one of a screen, a collection of lights, a projector, or a holographic display. In some examples, the display 512 is a touchscreen.

In some implementations, the display 512 visually outputs a pairing pattern 516. The pairing pattern 516 includes one or more shapes and/or colors that are detectable by the second medical device 504 or the intermediary device 506. In some implementations, the pairing pattern 516 is animated, such that it changes shape and/or color over time. The second medical device 504 or intermediary device 506, in some cases, pairs with the first medical device 502 based on detecting the pairing pattern 516.

According to some examples, the display 512 visually outputs a barcode 518. The barcode 518 includes one or more shapes that encode an alphanumeric code. For instance, the barcode 518 is a quick response (QR) code. In various cases, the second medical device 504 or intermediary device 506 pairs with the first medical device 502 based on detecting the barcode 518.

According to some implementations, the second medical device 504 and/or the intermediary device 506 include sensors configured to detect the light 510, the pairing pattern 516, and the barcode 518. For instance, the second medical device 504 or the intermediary device 506 includes a camera configured to capture an image and/or video depicting the light 510, the pairing pattern 516, or the barcode 518.

In some implementations, the display 512 visually outputs an input code 514. A user, for instance, pairs the first medical device 502 with the second medical device 504 or the intermediary device 506 by inputting the input code 514 into the second medical device 504 or the intermediary device 506.

In various cases, codes and/or patterns provided by the light 510, the input code 514, the pairing pattern 516, or the barcode 518 indicate a link key. For example, the input code 514 itself, or a code encoded by a pattern output by the light 510, the pairing pattern 516, or the barcode 518, is a link key. In some cases, the second medical device 504 or the intermediary device 506 stores data that associates a link key a pattern output by the first medical device 502, such that the second medical device 504 or the intermediary device 506 is configured to initiate pairing upon confirmation and/or detection of the light 510, the input code 514, the pairing pattern 516, or the barcode 518 output by the first medical device 502.

The display 512 optionally outputs a pairing element 520 that enables a user to select one or more devices detected by the first medical device 502. For example, the pairing element 520 includes a user-selectable drop-down menu that highlights a selected device. The user, for instance, selects a device by touching a shape in the drop-down menu that corresponds to the device. In some implementations, the first medical device 502 generates the pairing element 520 upon detecting beacons transmitted by the second medical device 504 and the intermediary device 506.

In various implementations, the first medical device 502 includes one or more input devices. For instance, the display 512 is physically integrated with one or more touch sensors configured to detect the user (or an object) touching the display 512. In some cases, the first medical device 502 includes a button 522 that receives an input signal from a user when the user presses the button 522. The input signal, for instance, causes the first medical device 502 to pair with a particular device, to emit a beacon, to unpair from a particular device, or initiate other actions described herein.

According to some cases, the first medical device 502, the second medical device 504, and the intermediary device 506 include output devices that indicate whether they are paired to another device. For example, the second medical device 504 includes a pairing indicator 524 that outputs a signal indicative of the second medical device 504 being paired with the first medical device 502 or the intermediary device 506. In some cases, the pairing indicator 524 is a light and/or display that outputs visual signals. For instance, the pairing indicator 514 outputs a red signal (or blinks) when the second medical device 504 is not paired with any device, and outputs a green signal (or does not blink) when the second medical device 504 is paired with the first medical device 502.

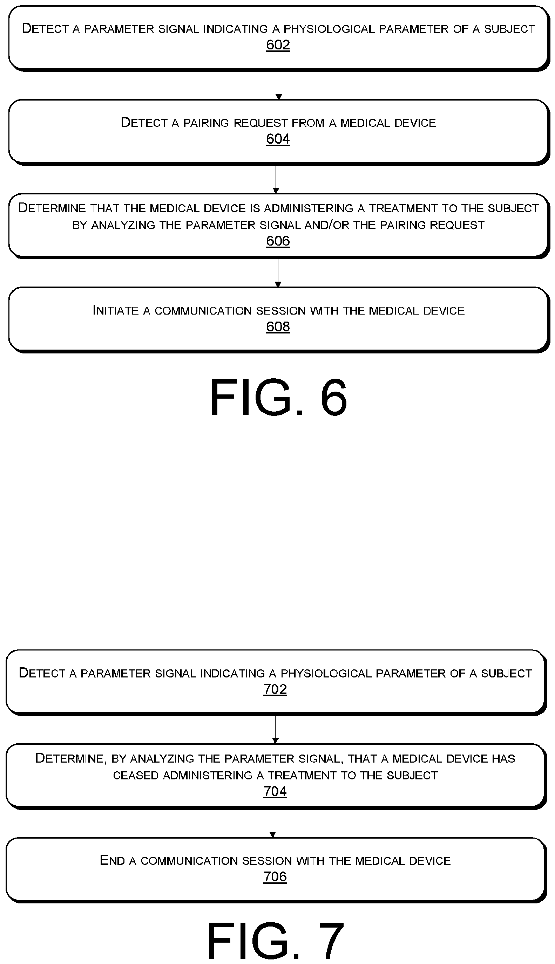

FIGS. 6 and 7 illustrate processes performed by one or more systems, devices, or entities described herein. For example, the processes illustrated in FIGS. 6 and 7 are implemented by an entity including at least one of the monitoring device 106, the monitoring device 202, the first medical device 502, the second medical device 506, any type of medical device described herein, or at least one processor configured to execute instructions.

FIG. 6 illustrates an example process 600 for pairing medical devices. At 602, the entity detects a parameter signal indicating a physiological parameter of a subject. In some implementations, the parameter signal is generated by at least one sensor. The sensor(s) is part of the entity and/or communicatively coupled to the entity. The physiological parameter, for example, is an ECG, an impedance, a force administered to the subject, a blood pressure, an airway parameter (e.g., a partial pressure of carbon dioxide, a partial pressure of oxygen, a capnograph, an end tidal gas parameter, a flow rate, etc.), a blood oxygenation (e.g., a pulse oximetry value, a regional oximetry value, etc.), an EEG, a temperature, a heart sound, a blood flow rate, a physiological geometry (e.g., a shape of a blood vessel, an inner ear shape, etc.), a heart rate, a pulse rate, or any combination thereof. In various cases, the parameter signal is sampled over time (e.g., at a particular sampling rate) and can be represented as multiple data points over time and/or a waveform.

At 604, the entity detects a pairing request from a medical device. In some implementations, the pairing request indicates a key (e.g., a link key, an encryption key, a decryption key, etc.). The pairing request, in some cases, identifies the medical device. For instance, the pairing request includes an identifier that uniquely identifies the medical device among multiple medical devices in a fleet of devices. In some implementations, the pairing request includes a treatment parameter. For example, the treatment parameter characterizes a treatment administered by the medical device to the subject. In some examples, the pairing request includes synchronization information. In some cases, the pairing request indicates a communication capability of the medical device.

At 606, the entity determines that the medical device is administering the treatment to the subject by analyzing the parameter signal and/or the pairing request. In some examples, entity identifies an artifact in the parameter signal that is indicative of the treatment. According to some cases, the medical device determines that the medical device is administering the treatment by analyzing the artifact in view of information in the pairing request.

At 608, the entity initiates a communication session with the medical device. In some implementations, the entity pairs with the medical device. In some cases, the entity engages in a handshake with the medical device. According to various examples, the entity transmits data to the medical device and/or receives data from the medical device. The data, for instance, is encrypted by the key and/or decrypted by the key. In some cases, the data includes at least one report, at least one instruction, or a combination thereof.

FIG. 7 illustrates an example process 700 for unpairing medical devices. At 702, the entity detects a parameter signal indicating a physiological parameter of a subject. In some implementations, the parameter signal is generated by at least one sensor. The sensor(s) is part of the entity and/or communicatively coupled to the entity. The physiological parameter, for example, is an ECG, an impedance, a force administered to the subject, a blood pressure, an airway parameter (e.g., a partial pressure of carbon dioxide, a partial pressure of oxygen, a capnograph, an end tidal gas parameter, a flow rate, etc.), a blood oxygenation (e.g., a pulse oximetry value, a regional oximetry value, etc.), an EEG, a temperature, a heart sound, a blood flow rate, a physiological geometry (e.g., a shape of a blood vessel, an inner ear shape, etc.), a heart rate, a pulse rate, or any combination thereof. In various cases, the parameter signal is sampled over time (e.g., at a particular sampling rate) and can be represented as multiple data points over time and/or a waveform.

At 704, the entity determines, by analyzing the parameter signal, that a medical device has ceased administering a treatment to the subject. For instance, the entity may have previously identified an artifact indicative of the treatment in the parameter signal. In some cases, the entity determines that the artifact is no longer present in the parameter signal.

At 706, the entity ends a communication session with the medical device. For example, the entity may refrain from transmitting data to the medical device and/or receiving data from the medical device. In some implementations, the entity unpairs from the medical device. For instance, the entity transmits an unpairing request to the medical device.

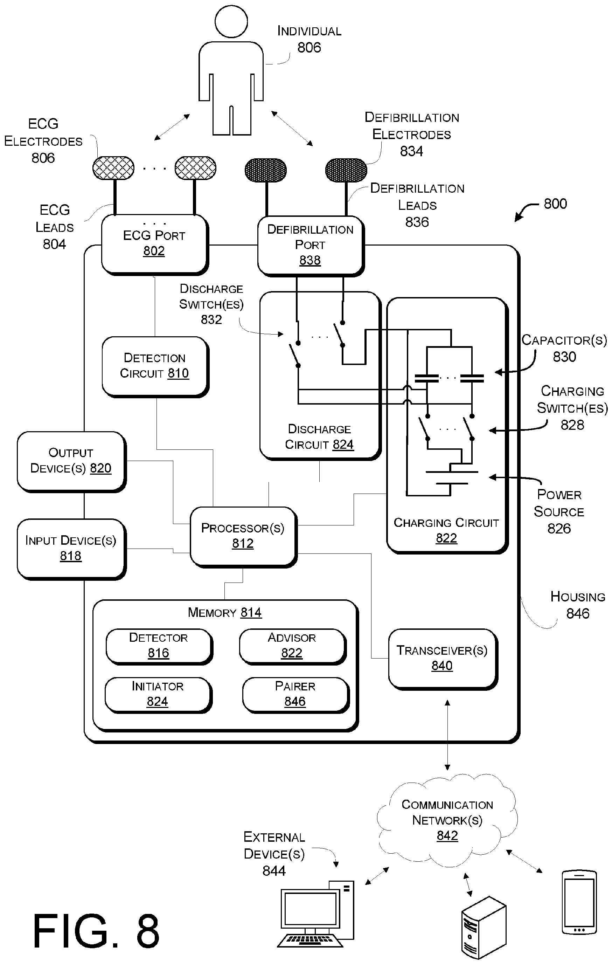

FIG. 8 illustrates an example of an external defibrillator 800 configured to perform various functions described herein. For example, the external defibrillator 800 is the monitoring device 106, the first treating device 110, the second treating device 114, the monitoring device 202, the treating device 204, the first medical device 502, the second medical device 504, or the intermediary device 506 described above with reference to FIGS. 1A to 5.

The external defibrillator 800 includes an electrocardiogram (ECG) port 802 connected to multiple ECG leads 804. In some cases, the ECG leads 804 are removeable from the ECG port 802. For instance, the ECG leads 804 are plugged into the ECG port 802. The ECG leads 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 808 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG leads 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage (or current) across a pair of the ECG electrodes 806 and detects a resultant current (or voltage) between the pair of the ECG electrodes 806. The impedance is generated based on the applied signal (voltage or current) and the resultant signal (current or voltage). In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance."

The detection circuit 810 provides the ECG signal and/or the impedance signal one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 812 is volatile (such as random access memory (RAM)), non-volatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and ventricular tachycardia (V-Tach). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. In some examples, the processor(s) 812 executing the initiator 824 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

The processor(s) 812 is operably connected to a charging circuit 822 and a discharge circuit 824. In various implementations, the charging circuit 822 includes a power source 826, one or more charging switches 828, and one or more capacitors 830. The power source 826 includes, for instance, a battery. The processor(s) 812 initiates a defibrillation shock by causing the power source 826 to charge at least one capacitor among the capacitor(s) 830. For example, the processor(s) 812 activates at least one of the charging switch(es) 828 in the charging circuit 822 to complete a first circuit connecting the power source 826 and the capacitor to be charged. Then, the processor(s) 812 causes the discharge circuit 824 to discharge energy stored in the charged capacitor across a pair of defibrillation electrodes 830, which are in contact with the individual 808. For example, the processor(s) 812 deactivates the charging switch(es) 828 completing the first circuit between the capacitor(s) 830 and the power source 826, and activates one or more discharge switches 832 completing a second circuit connecting the charged capacitor 830 and at least a portion of the individual 808 disposed between defibrillation electrodes 834.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation leads 836. The defibrillation leads 836 are connected to a defibrillation port 838, in implementations. According to various examples, the defibrillation leads 836 are removable from the defibrillation port 838. For example, the defibrillation leads 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 840 includes other wireless modems, such as a modem for engaging in WI-FI^{®}, WIGIG^{®}, WIMAX^{®}, BLUETOOTH^{®}, or infrared communication over the communication network(s) 842.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis.

In some cases, the external device(s) 844 include one or more medical devices. According to various implementations, the memory 814 further includes a pairer 846 which, when executed by the processor(s) 812, causes the processor(s) 812 to pair the defibrillator 800 with the external device(s) 844 and/or to unpair the defibrillator 800 from the external device(s) 844. In some implementations, the processor(s) 812, when executing the pairer 846, detects a treatment being administered to the individual 806 by the external device(s) 844 by analyzing a parameter signal including the ECG detected by the detection circuit 810 and/or another type of physiological parameter detected by the input device(s) 818. In some implementations, the processor(s) further identify the treatment based on a signal received by the transceiver(s) 840. The processor(s) 812, for instance, cause the transceiver(s) 840 to exchange data with the external device(s) 844 over an encrypted communication channel based on determining that the treatment is being administered by the external device(s) 844. In some examples, the pairer 846, when executed by the processor(s) 812, further causes the processor(s) 812 to identify when the treatment is no longer being administered to the individual 806 by the external device(s) 844 by analyzing the parameter signal. In response, the processor(s) 812 cause the transceiver(s) 840 to refrain from exchanging data with the external device(s) 844 over the encrypted communication channel.

In some cases, the processor(s) 812, when executing the pairer 846, generates one or more pairing requests and/or responses that are transmitted, by the transceiver(s) 840, to the external device(s) 844. In some examples, the processor(s) 812, when executing the pairer 846, analyzes one or more pairing requests and/or pairing responses that are received, by the transceiver(s) 840 from the external device(s) 844. In various cases, the pairer 846 causes the processor(s) 812 to initiate pairing and/or unpairing with the external device(s) 844. For example, the pairer 846 causes the processor(s) 812 to generate and/or encrypt data that the transceiver(s) 840 transmit over one or more communication channels.

In various implementations, the external defibrillator 800 also includes a housing 846 that at least partially encloses other elements of the external defibrillator 800. For example, the housing 846 encloses the detection circuit 810, the processor(s) 812, the memory 814, the charging circuit 822, the transceiver(s) 840, or any combination thereof. In some cases, the input device(s) 818 and output device(s) 820 extend from an interior space at least partially surrounded by the housing 846 through a wall of the housing 846. In various examples, the housing 846 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, discharges the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

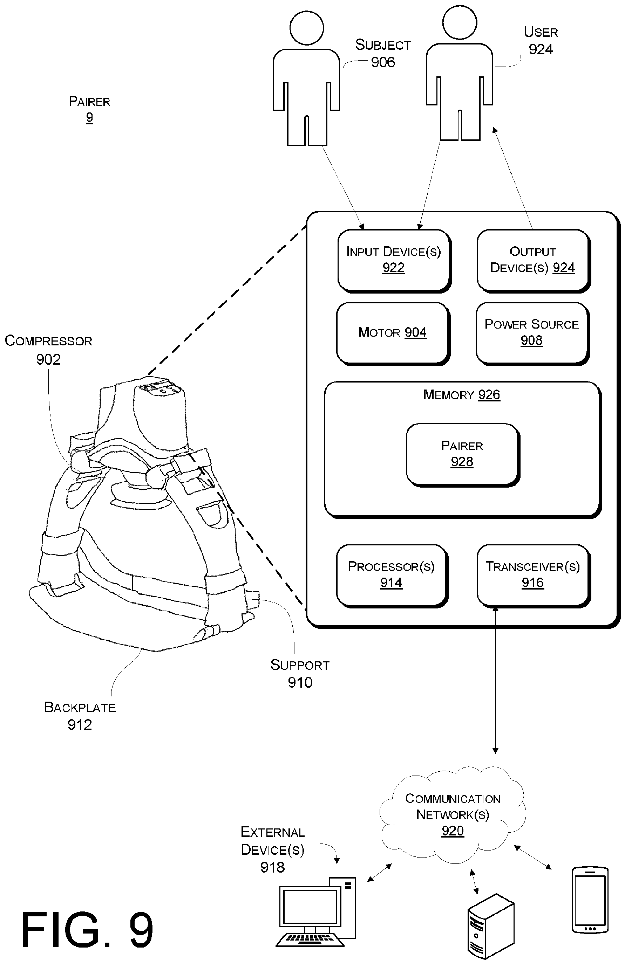

FIG. 9 illustrates a chest compression device 900 configured to perform various functions described herein. For example, the chest compression device 900 is the monitoring device 106, the first treating device 110, the second treating device 114, the monitoring device 202, the treating device 204, the first medical device 502, the second medical device 504, or the intermediary device 506 described above with reference to FIGS. 1A to 5.

In various implementations, the chest compression device 900 includes a compressor 902 that is operatively coupled to a motor 904. The compressor 902 physically administers a force to the chest of a subject 906 that compresses the chest of the subject 906. In some examples, the compressor 902 includes at least one piston that periodically moves between two positions (e.g., a compressed position and a release position) at a compression frequency. For example, when the piston is positioned on the chest of the subject 906, the piston compresses the chest when the piston is moved into the compressed position. A suction cup may be positioned on a tip of the piston, such that the suction cup contacts the chest of the subject 906 during operation. In various cases, the compressor 902 includes a band that periodically tightens to a first tension and loosens to a second tension at a compression frequency. For instance, when the band is disposed around the chest of the subject 906, the band compresses the chest when the band tightens.

The motor 904 is configured to convert electrical energy stored in a power source 908 into mechanical energy that moves and/or tightens the compressor 902, thereby causing the compressor 902 to administer the force to the chest of the subject 906. In various implementations, the power source 908 is portable. For instance, the power source 908 includes at least one rechargeable (e.g., lithium-ion) battery. In some cases, the power source 908 supplies electrical energy to one or more elements of the chest compression device 900 described herein.

In various cases, the chest compression device 900 includes a support 910 that is physically coupled to the compressor 902, such that the compressor 902 maintains a position relative to the subject 906 during operation. In some implementations, the support 910 is physically coupled to a backplate 912, cot, or other external structure with a fixed position relative to the subject 906. According to some cases, the support 910 is physically coupled to a portion of the subject 906, such as wrists of the subject 906.

The operation of the chest compression device 900 may be controlled by at least one processor 914. In various implementations, the motor 906 is communicatively coupled to the processor(s) 914. Specifically, the processor(s) 914 is configured to output a control signal to the motor 906 that causes the motor 906 to actuate the compressor 902. For instance, the motor 906 causes the compressor 902 to administer the compressions to the subject 906 based on the control signal. In some cases, the control signal indicates one or more treatment parameters of the compressions. Examples of treatment parameters include a frequency, timing, depth, force, position, velocity, and acceleration of the compressor 902 administering the compressions. According to various cases, the control signal causes the motor 906 to cease compressions.

In various implementations, the chest compression device 900 includes at least one transceiver 916 configured to communicate with at least one external device 918 over one or more communication networks 920. Any communication network described herein can be included in the communication network(s) 920 illustrated in FIG. 9. The external device(s) 918, for example, includes at least one of a monitor-defibrillator, an AED, an ECMO device, a ventilation device, a patient monitor, a mobile phone, a server, or a computing device. In some implementations, the transceiver(s) 916 is configured to communicate with the external device(s) 918 by transmitting and/or receiving signals wirelessly. For example, the transceiver(s) 916 includes a NIC, a network adapter, a LAN adapter, or a physical, virtual, or logical address to connect to the various external devices and/or systems. In various examples, the transceiver(s) 916 includes any sort of wireless transceivers capable of engaging in wireless communication (e.g., RF communication). For example, the communication network(s) 920 includes one or more wireless networks that include a 3GPP network, such as an LTE RAN (e.g., over one or more LTE bands), an NR RAN (e.g., over one or more NR bands), or a combination thereof. In some cases, the transceiver(s) 916 includes other wireless modems, such as a modem for engaging in WI-FI^{®}, WIGIG^{®}, WIMAX^{®}, BLUETOOTH^{®}, or infrared communication over the communication network(s) 920. The signals, in various cases, encode data in the form of data packets, datagrams, or the like. In some cases, the signals are transmitted as compressions are being administered by the chest compression device 900 (e.g., for real-time feedback by the external device(s) 918), after compressions are administered by the chest compression device 900 (e.g., for post-event review at the external device 918), or a combination thereof.

In various cases, the processor(s) 914 generates the control signal based on data encoded in the signals received from the external device(s) 918. For instance, the signals include an instruction to initiate the compressions, and the processor(s) 914 instructs the motor 906 to begin actuating the compressor 902 in accordance with the signals.

In some cases, the chest compression device 900 includes at least one input device 922. In various examples, the input device(s) 922 is configured to receive an input signal from a user 924, who may be a rescuer treating the subject 906. Examples of the input device(s) 922 include, for instance, at a keypad, a cursor control, a touch-sensitive display, a voice input device (e.g., a microphone), a haptic feedback device (e.g., a gyroscope), or any combination thereof. In various implementations, the processor(s) 914 generate the control signal based on the input signal. For instance, the processor(s) 914 generate the control signal to adjust a frequency of the compressions based on the chest compression device 900 detecting a selection by the user 924 of a user interface element displayed on a touchscreen or detecting the user 924 pressing a button integrated with an external housing of the chest compression device 900.

According to some examples, the input device(s) 922 include one or more sensors. The sensor(s), for example, is configured to detect a physiological parameter of the subject 906. In some implementations, the sensor(s) is configured to detect a state parameter of the chest compression device 900, such as a position of the compressor 902 with respect to the subject 906 or the backplate 912, a force administered by the compressor 902 on the subject 906, a force administered onto the backplate 922 by the body of the subject 906 during a compression, or the like. According to some implementations, the signals transmitted by the transceiver(s) 916 indicate the physiological parameter(s) and/or the state parameter(s).

The chest compression device 900 further includes at least one output device 924, in various implementations. Examples of the output device(s) 924 include, for instance, least one of a display (e.g., a projector, an LED screen, etc.), a speaker, a haptic output device, a printer, or any combination thereof. In some implementations, the output device(s) 924 include a screen configured to display various parameters detected by and/or reported to the chest compression device 900, a charge level of the power source 908, a timer indicating a time since compressions were initiated or paused, and other relevant information.

The chest compression device 900 further includes memory 926. In various implementations, the memory 926 is volatile (such as random access memory (RAM)), non-volatile (such as read only memory (ROM), flash memory, etc.) or some combination of the two. The memory 926 stores instructions that, when executed by the processor(s) 914, causes the processor(s) 914 to perform various operations. In various examples, the memory 926 stores methods, threads, processes, applications, objects, modules, any other sort of executable instruction, or a combination thereof. In some cases, the memory 926 stores files, databases, or a combination thereof. In some examples, the memory 926 includes, but is not limited to, RAM, ROM, EEPROM, flash memory, or any other memory technology. In some examples, the memory 926 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 926 stores instructions, programs, threads, objects, data, or any combination thereof, that cause the processor(s) 914 to perform various functions. In various cases, the memory 926 stores one or more parameters that are detected by the chest compression device 900 and/or reported to the chest compression device 900.

In implementations of the present disclosure, the memory 926 also stores instructions for executing a pairer 928. In some cases, the processor(s) 914, when executing the pairer 928, generates one or more pairing requests and/or responses that are transmitted, by the transceiver(s) 916, to the external device(s) 918. In some examples, the processor(s) 914, when executing the pairer 928, analyzes one or more pairing requests and/or pairing responses that are received, by the transceiver(s) 916), from the external device(s) 918. In various cases, the pairer 928 causes the processor(s) 914 to initiate pairing and/or unpairing with the external device(s) 918. For example, the pairer 928 causes the processor(s) 914 to generate and/or encrypt data that the transceiver(s) 916 transmit over one or more communication channels.

### EXAMPLE CLAUSES

1. A system, including: a mechanical chest compression device configured to administer chest compressions to a subject and to broadcast a first wireless signal indicating a frequency or a timing of the chest compressions; and a monitor-defibrillator including: a detection circuit configured to detect a parameter signal including an electrocardiogram (ECG) of the subject; a transceiver configured to receive the first wireless signal from the mechanical chest compression device and to transmit a second wireless signal to the mechanical chest compression device; a discharge circuit configured to output an electrical shock to the subject; and a processor configured to: determine that the mechanical chest compression device is administering the chest compressions to the subject by analyzing the parameter signal in view of the frequency or the timing of the chest compressions; in response to determining that the mechanical chest compression device is administering the chest compressions to the subject, cause the transceiver to initiate a wireless communication channel with the mechanical chest compression device; determine that the subject is experiencing ventricular fibrillation (VF) by analyzing the parameter signal; and in response to determining that the subject is experiencing VF: cause the transceiver to transmit the second wireless signal to the mechanical chest compression device over the wireless communication channel, the second wireless signal including an instruction to cease administering the chest compressions at a predetermined time; and cause the discharge circuit to output the electrical shock to the subject at the predetermined time.
2. The system of clause 1, wherein the processor is further configured to: identify a comb filter characterized by the frequency or the timing of the chest compressions; remove a chest compression artifact from data representing the parameter signal by applying the comb filter to the data, and wherein determining that the subject is experiencing VF by analyzing the parameter signal includes analyzing the data representing the parameter signal in response to applying the comb filter to the data.
3. The system of clause 1 or 2, wherein the first wireless signal further includes an identifier of the mechanical chest compression device and a communication capability of the mechanical chest compression device, and wherein the processor is configured to cause the transceiver to initiate the wireless communication channel with the mechanical chest compression device by: causing the transceiver to transmit, to the mechanical chest compression device, a pairing request including the identifier, the pairing request being consistent with the communication capability of the mechanical chest compression device; and causing the transceiver to receive, from the mechanical chest compression device, a pairing response indicating that the wireless communication channel has been established.
4. A first medical device, including: a sensor configured to detect a parameter signal indicating a physiological parameter of a subject; a transceiver; and a processor configured to: determine, by analyzing the parameter signal, that a second medical device is administering a treatment to the subject; in response to determining that the second medical device is administering the treatment to the subject, pair the first medical device with the second medical device; and in response to pairing the first medical device with the second medical device, cause the transceiver to transmit, over a wireless channel, data to the second medical device or to receive, over the wireless channel, data from the second medical device.
5. The first medical device of clause 4, wherein the sensor includes an electrocardiogram (ECG) sensor or an impedance sensor, and wherein the treatment includes chest compressions.
6. The first medical device of clause 5, wherein the processor is configured to determine, by analyzing the parameter signal, that the second medical device is administering the treatment to the subject by: detecting, in the parameter signal, an artifact indicative of the chest compressions being administered to the subject by the second medical device.
7. The first medical device of any of clauses 4 to 6, further including: an input device configured to receive an input signal indicating the second medical device, wherein the processor is configured to pair the first device with the second medical device in response to the input device receiving the input signal.
8. The first medical device of any of clauses 4 to 7, wherein the transceiver is configured to receive a signal that includes pairing information, and wherein the processor is configured to pair the first device with the second device by utilizing the pairing information.
9. The first medical device of clause 8, wherein the transceiver receives the signal from the second medical device, a passkey device, a cot, or an accessory device.
10. The first medical device of any of clauses 4 to 9, wherein the transceiver is configured to receive, from the second medical device over the wireless channel, a signal that indicates a treatment parameter, and wherein the processor is configured to determine, by analyzing the parameter signal, that the second medical device is administering the treatment to the subject by: determining, by analyzing the parameter signal, that the treatment is consistent with the treatment parameter.
11. The first medical device of any of clauses 4 to 10, the treatment being a first treatment, the first medical device further including: memory storing clock data, wherein the processor is configured to cause the transceiver to transmit, to the second medical device over the wireless channel, a synchronization message indicative of the clock data; and causing the transceiver to transmit, to the second medical device over the wireless channel, an instruction to perform a second treatment at a time that is in reference to the clock data.
12. The first medical device of any of clauses 4 to 11, the treatment being a first treatment, wherein the processor is configured to cause the transceiver to periodically transmit, to the second medical device over the wireless channel, first data packets indicating the physiological parameter of the subject; and cause the transceiver to transmit, to the second medical device over the wireless channel, a second data packet including an instruction to perform a second treatment.
13. A method, including: detecting a signal indicating a physiological parameter of a subject; determining, by analyzing the signal, that a second medical device is administering a treatment to the subject; in response to determining that the second medical device is administering the treatment to the subject, pairing a first medical device with the second medical device; and in response to pairing the first medical device with the second medical device, transmitting data to the second medical device over a wireless channel.
14. The method of clause 13, wherein the physiological parameter includes an electrocardiogram (ECG), an impedance, a force, a blood pressure, a partial pressure of carbon dioxide, a partial pressure of oxygen, a capnograph, an end tidal gas parameter, a flow rate, a blood oxygenation, an EEG, a temperature, a heart sound, a blood flow rate, a physiological geometry, a heart rate, or a pulse rate of the subject.
15. The method of clause 13 or 14, wherein transmitting the data to the second medical device over the wireless channel includes transmitting an electromagnetic signal encoded with the data.
16. The method of any of clauses 13 to 15, wherein determining, by analyzing the signal, that the second medical device is administering the treatment to the subject includes: detecting, in the signal, an artifact indicative of the treatment being administered to the subject by the second medical device.
17. The method of any of clauses 13 to 16, the signal being a first signal, the method further including: receiving a second signal including pairing information, wherein pairing the first medical device with the second device includes: identifying a link key based on the pairing information, and wherein transmitting data to the second medical device over the wireless channel includes encrypting the data using the link key.
18. The method of clause 17, wherein the second signal is received from the second medical device, a passkey device, a cot, or an accessory device.
19. The method of any of clauses 13 to 18, the signal being a first signal, the method further including: receiving a second signal including a treatment parameter, wherein determining, by analyzing the first signal, that the second medical device is administering the treatment to the subject includes: determining, by analyzing the signal, that the treatment is consistent with the treatment parameter.
20. The method of any of clauses 13 to 19, the treatment being a first treatment, wherein transmitting data over the wireless channel includes: periodically transmitting, to the second medical device over the wireless channel, first data packets indicating the physiological parameter of the subject; and transmitting, to the second medical device over the wireless channel, a second data packet including an instruction to perform a second treatment.
21. A system, including: a mechanical chest compression device configured to administer compressions, to broadcast a first wireless signal including a treatment parameter characterizing the compressions, and to receive a second wireless signal over a communication channel; and a monitor-defibrillator including: a detection circuit configured to detect a parameter signal including an electrocardiogram (ECG) of a subject; a transceiver configured to receive the first wireless signal from the mechanical chest compression device and to transmit the second wireless signal to the mechanical chest compression device over the communication channel; a discharge circuit configured to output an electrical shock to the subject; and a processor configured to: identify a filter characterized by the treatment parameter; remove a compression artifact from first data representing the parameter signal detected during a first time period by applying the filter to the first data; determine that the compression artifact is omitted from the parameter signal during a second time period; determine that the treatment parameter indicates that the mechanical chest compression device is administering the compressions during the second time period; and in response to determining that the compression artifact is omitted from the parameter signal during the second time period and to determining that the treatment parameter indicates that the mechanical chest compression device is administering the compressions during the second time period: cause the transceiver to end the communication channel with the mechanical chest compression device; refrain from applying the filter to second data representing the parameter signal detected during the second time period; determine that the subject is experiencing ventricular fibrillation (VF) by analyzing second data; and in response to determining that the subject is experiencing VF, cause the discharge circuit to output the electrical shock to the subject.
22. The system of clause 21, wherein the treatment parameter indicates a frequency of the compressions, and wherein the filter includes a comb filter including a first notch at the frequency of the compressions and a second notch at a harmonic of the frequency of the compressions.
23. The system of clause 21 or 22, wherein the processor is configured to cause the transceiver to end the communication channel with the mechanical chest compression device by: causing the transceiver to transmit a third wireless signal to the mechanical chest compression device, the third wireless signal indicating an unpairing instruction.
24. A first medical device, including: a sensor configured to detect a parameter signal indicating a physiological parameter of a subject, the subject receiving a treatment from a second medical device during a first time period; a transceiver configured to receive a wireless signal from the second medical device over a communication channel; and a processor configured to: determine that the subject is not receiving the treatment during a second time period by determining that an artifact associated with the treatment is omitted from the parameter signal during the second time period; and in response to determining that the subject is not receiving the treatment during the second time period, causing the transceiver to end the communication channel.
25. The first medical device of clause 24, wherein the sensor includes an electrocardiogram (ECG) sensor or an impedance sensor.
26. The first medical device of clause 24 or 25, wherein the treatment includes chest compressions, pacing, or an electrical shock.
27. The first medical device of any of clauses 24 to 26, the treatment being a first treatment, wherein the wireless signal indicates a treatment parameter characterizing the first treatment administered by the second medical device, and wherein the processor is further configured to: generate a filter characterized by the treatment parameter; remove an artifact from data representing the parameter signal during the first time period by applying the filter to the data; in response to removing the artifact from the data representing the parameter signal during the first time period, generate a recommendation for a second treatment by analyzing the data representing the parameter signal during the first time period; and output the recommendation.
28. The first medical device of any of clauses 24 to 27, wherein the recommendation is to administer an electrical shock, administer chest compressions, or pause the chest compressions.
29. The first medical device of clause 27 or 28, the recommendation being a first recommendation, the data being first data, wherein the processor is further configured to: generate a second recommendation for a third treatment by analyzing second data representing the parameter signal during the second time period without applying the filter to the second data; and output the second recommendation.
30. The first medical device of any of clauses 24 to 29, wherein the wireless signal indicates that the second medical device is administering the treatment during the first time period and the second time period.
31. The first medical device of any of clauses 24 to 30, wherein the first medical device includes a mechanical chest compression device or an external defibrillator.
32. The first medical device of any of clauses 24 to 31, the wireless signal being a first wireless signal, wherein the processor is configured to cause the transceiver to end the communication channel by: causing the transceiver to transmit, to the second medical device, a second wireless signal indicating an unpairing instruction.
33. A method performed by a first medical device, the method including: detecting a parameter signal indicating a physiological parameter of a subject, the subject receiving a treatment from a second medical device during a first time period; receiving a wireless signal from the second medical device over a communication channel; determining that the subject is not receiving the treatment during a second time period by determining that an artifact associated with the treatment is omitted from the parameter signal during the second time period; and in response to determining that the subject is not receiving the treatment during the second time period, ending the communication channel.
34. The method of clause 33, wherein the physiological parameter includes an electrocardiogram (ECG) or an impedance.
35. The method of clause 33 or 34, wherein the treatment includes chest compressions, an electrical shock, or pacing.
36. The method of any of clauses 33 to 35, the treatment being a first treatment, wherein the wireless signal indicates a treatment parameter characterizing the first treatment administered by the second medical device, and wherein the method further includes: generating a filter characterized by the treatment parameter; removing an artifact from data representing the parameter signal during the first time period by applying the filter to the data; in response to removing the artifact from the data representing the parameter signal during the first time period, generating a recommendation for a second treatment by analyzing the data representing the parameter signal during the first time period; and outputting the recommendation.
37. The method of clause 36, wherein the recommendation is to administer an electrical shock, administer chest compressions, or pause the chest compressions.
38. The method of clause 36 or 37, the recommendation being a first recommendation, the data being first data, wherein the method further includes: generating a second recommendation for a third treatment by analyzing second data representing the parameter signal during the second time period without applying the filter to the second data; and outputting the second recommendation.
39. The method of any of clauses 33 to 38, wherein the wireless signal indicates that the second medical device is administering the treatment during the first time period and the second time period.
40. The method of any of clauses 33 to 39, the wireless signal being a first wireless signal, wherein ending the communication channel includes: transmitting, to the second medical device, a second wireless signal indicating an unpairing instruction.

### CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11 % of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1 % of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A first medical device, comprising:
a sensor configured to detect a parameter signal indicating a physiological parameter of a subject;
a transceiver; and
a processor configured to:
determine, by analyzing the parameter signal, that a second medical device is administering a treatment to the subject;
in response to determining that the second medical device is administering the treatment to the subject, pair the first medical device with the second medical device; and
in response to pairing the first medical device with the second medical device, cause the transceiver to transmit, over a wireless channel, data to the second medical device or to receive, over the wireless channel, data from the second medical device.

2. The first medical device of claim 1, wherein the sensor comprises an electrocardiogram (ECG) sensor or an impedance sensor, and
wherein the treatment comprises chest compressions.

3. The first medical device of claim 2, wherein the processor is configured to determine, by analyzing the parameter signal, that the second medical device is administering the treatment to the subject by:
detecting, in the parameter signal, an artifact indicative of the chest compressions being administered to the subject by the second medical device.

4. The first medical device of any of claims 1-3, further comprising:
an input device configured to receive an input signal indicating the second medical device,
wherein the processor is configured to pair the first device with the second medical device in response to the input device receiving the input signal.

5. The first medical device of any of claims 1-4, wherein the transceiver is configured to receive a signal that comprises pairing information, and
wherein the processor is configured to pair the first device with the second device by utilizing the pairing information,
wherein optionally the transceiver receives the signal from the second medical device, a passkey device, a cot, or an accessory device.

6. The first medical device of any of claims 1-5, wherein the transceiver is configured to receive, from the second medical device over the wireless channel, a signal that indicates a treatment parameter, and
wherein the processor is configured to determine, by analyzing the parameter signal, that the second medical device is administering the treatment to the subject by:
determining, by analyzing the parameter signal, that the treatment is consistent with the treatment parameter.

7. The first medical device of any of claims 1-6, the treatment being a first treatment, the first medical device further comprising:
memory storing clock data,
wherein the processor is configured to cause the transceiver to transmit, to the second medical device over the wireless channel, a synchronization message indicative of the clock data; and
causing the transceiver to transmit, to the second medical device over the wireless channel, an instruction to perform a second treatment at a time that is in reference to the clock data.

8. The first medical device of any of claims 1-7, the treatment being a first treatment, wherein the processor is configured to cause the transceiver to periodically transmit, to the second medical device over the wireless channel, first data packets indicating the physiological parameter of the subject; and
cause the transceiver to transmit, to the second medical device over the wireless channel, a second data packet comprising an instruction to perform a second treatment.

9. A method, comprising:
detecting a signal indicating a physiological parameter of a subject;
determining, by analyzing the signal, that a second medical device is administering a treatment to the subject;
in response to determining that the second medical device is administering the treatment to the subject, pairing a first medical device with the second medical device; and
in response to pairing the first medical device with the second medical device, transmitting data to the second medical device over a wireless channel.

10. The method of claim 9, wherein the physiological parameter comprises an electrocardiogram (ECG), an impedance, a force, a blood pressure, a partial pressure of carbon dioxide, a partial pressure of oxygen, a capnograph, an end tidal gas parameter, a flow rate, a blood oxygenation, an EEG, a temperature, a heart sound, a blood flow rate, a physiological geometry, a heart rate, or a pulse rate of the subject.

11. The method of claim 9 or 10, wherein transmitting the data to the second medical device over the wireless channel comprises transmitting an electromagnetic signal encoded with the data; and/or
wherein determining, by analyzing the signal, that the second medical device is administering the treatment to the subject comprises:
detecting, in the signal, an artifact indicative of the treatment being administered to the subject by the second medical device.

12. The method of any of claims 9-11, the signal being a first signal, the method further comprising:
receiving a second signal comprising pairing information,
wherein pairing the first medical device with the second device comprises:
identifying a link key based on the pairing information, and
wherein transmitting data to the second medical device over the wireless channel comprises encrypting the data using the link key,
wherein optionally the second signal is received from the second medical device, a passkey device, a cot, or an accessory device.

13. The method of any of claims 9-12, the signal being a first signal, the method further comprising:
receiving a second signal comprising a treatment parameter,
wherein determining, by analyzing the first signal, that the second medical device is administering the treatment to the subject comprises:
determining, by analyzing the signal, that the treatment is consistent with the treatment parameter.

14. The method of any of claims 9-13, the treatment being a first treatment, wherein transmitting data over the wireless channel comprises:
periodically transmitting, to the second medical device over the wireless channel, first data packets indicating the physiological parameter of the subject; and
transmitting, to the second medical device over the wireless channel, a second data packet comprising an instruction to perform a second treatment.

15. A system, comprising:
a mechanical chest compression device configured to administer chest compressions to a subject and to broadcast a first wireless signal indicating a frequency or a timing of the chest compressions; and
a first medical device according to any of claims 1-8 wherein the first medical device comprises a monitor-defibrillator, wherein:
the sensor comprises a detection circuit configured to detect a parameter signal comprising an electrocardiogram (ECG) of the subject;
the transceiver is configured to receive the first wireless signal from the mechanical chest compression device and to transmit a second wireless signal to the mechanical chest compression device;
the processor is configured to:
determine that the mechanical chest compression device is administering the chest compressions to the subject by analyzing the parameter signal in view of the frequency or the timing of the chest compressions;
in response to determining that the mechanical chest compression device is administering the chest compressions to the subject, cause the transceiver to initiate a wireless communication channel with the mechanical chest compression device;
determine that the subject is experiencing ventricular fibrillation (VF) by analyzing the parameter signal; and
in response to determining that the subject is experiencing VF:
cause the transceiver to transmit the second wireless signal to the mechanical chest compression device over the wireless communication channel, the second wireless signal comprising an instruction to cease administering the chest compressions at a predetermined time;
wherein the monitor-defibrillator comprises a discharge circuit configured to output an electrical shock to the subject; and
the processor is configured to cause the discharge circuit to output the electrical shock to the subject at the predetermined time.
